# EUROPÄISCHE PATENTSCHRIFT

(11) **EP 3 490 647 B1**
(45) Veröffentlichungstag und Bekanntmachung des Hinweises auf die Patenterteilung: **06.10.2021**
(21) Anmeldenummer: 17745963.3
(22) Anmeldetag: 26.07.2017
(51) Int. Cl.: A61M 5/32, A61M 5/20, A61M 5/24

(54) **INJEKTIONSVORRICHTUNG MIT ÄUSSERER KAPPE MIT NADELSCHUTZKAPPENENTFERNERELEMENT UND VERFAHREN ZUM MONTIEREN EINER INJEKTIONSVORRICHTUNG**
INJECTION DEVICE WITH OUTER CAP WITH NEEDLE CAP REMOVER ELEMENT AND METHOD TO ASSEMBLE AN INJECTION DEVICE
DISPOSITIF D'INJECTION AVEC UN CAPUCHON EXTERNE AVEC UN ÉLÉMENT POUR DÉCAPOUCHONNER UN CAPUCHON D'UNE AIGUILLE ET UNE MÉTHODE D'ASSEMBLAGE D'UNE DISPOSITIF D'INJECTION

(30) Priorität: 28.07.2016 CH 9892016; 08.09.2016 CH 11662016
(43) Veröffentlichungstag der Anmeldung: 05.06.2019
(73) Patentinhaber: Ypsomed AG, 3401 Burgdorf (CH)
(72) Erfinder: GROETZBACH, Felix, 4563 Gerlafingen (CH); TSCHIRREN, Markus, 3400 Burgdorf (CH); BURREN, Stefan, 3150 Schwarzenburg (CH); KLÖTZLI, Urs, 3400 Burgdorf (CH); STETTLER, Peter, 3423 Ersigen (CH); LORETZ, Benjamin, 4533 Riedholz (CH)
(74) Vertreter: Kleiner, Stefan
(86) Internationale Anmeldenummer: PCT/CH2017/000074
(87) Internationale Veröffentlichungsnummer: WO 2018/018167

(56) Entgegenhaltungen:
- EP-A1- 2 878 321
- EP-A1- 2 878 322
- EP-A1- 2 923 716
- WO-A1-2010/136076
- FR-A1- 3 011 186
- US-A1- 2010 016 795

## Beschreibung

Die Erfindung betrifft eine Injektionsvorrichtung zur Verabreichung eines flüssigen Produkts, insbesondere eines Medikaments. Ferner betrifft die Erfindung ein Verfahren zum Montieren und/oder Vorbereiten einer Injektionsvorrichtung für die Verabreichung eines Produkts.

Der Begriff "Medikament" umfasst hier jede fliessfähige medizinische Formulierung, welche geeignet ist zur kontrollierten Verabreichung durch ein Mittel wie eine Kanüle oder Hohlnadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen und weitere Substanzen sowohl in fester (suspendierter) oder flüssiger Form aber auch Polysaccaride, Vaccine, DNS oder RNS oder Oglionukleotide, Antikörper oder Teile von Antikörpern sowie geeignete Basis-, Hilfs- und Trägerstoffe.

Aus dem Stand der Technik sind Injektionsvorrichtungen bekannt, in denen eine Fertigspritze angeordnet ist. Die Fertigspritze weist eine Injektionsnadel auf, die unlösbar mit der Fertigspritze verbunden ist und über die ein in der Fertigspritze enthaltenes Medikament ausgegeben werden kann. Um die Injektionsnadel und das Medikament der Fertigspritze steril zu halten, wird die Injektionsnadel von einer an der Fertigspritze befestigten Nadelschutzkappe umschlossen und in Bezug auf die Umgebung steril abgedichtet. Solche Nadelschutzkappen können z.B. als sogenannte soft needle shield (SNS) oder als rigid needle shield (RNS) ausgestaltet sein. Ein soft needle shield (SNS) besteht aus einem elastomeren Teil, welches die Injektionsnadel umgibt. Ein rigid needle shield (RNS) weist mehrere Teile auf, insbesondere ein elastomeres kappenförmiges Teil und ein aus einem festen, d.h. nicht-elastomeren Kunststoff hergestellten hülsenförmiges Teil, welches das elastomere Teil aufnimmt und damit im Wesentlichen unlösbar verbunden ist.

Bei der Handhabung der Fertigspritze besteht die Gefahr, dass durch eine Krafteinwirkung auf die Nadelschutzkappe die Sterilität der Injektionsnadel und des Medikaments gefährdet wird. Dies kann insbesondere während des Montageprozesses der Injektionsvorrichtung, insbesondere wenn die Fertigspritze in ihren dafür vorgesehenen Spritzenhalter der Injektionsvorrichtung eingesetzt wird, auftreten. Das Einfügen der Fertigspritze in die Injektionsvorrichtung ist daher aus Sicht der Sterilität der Injektionsnadel und des Medikaments ein Schritt, dem besondere Aufmerksamkeit gilt. Aus der WO 2010/136076 A1, der FR 3011186 A1, der US 9,339,610 B2, der WO 2015/144871 A1 und US 2016/0243315 A1 ist es bekannt, dass beim Abziehen eines kappenförmigen Abziehelements, das auch als Kappe bezeichnet wird, am distalen Ende der Injektionsvorrichtung angebracht ist und das distale Ende der Injektionsvorrichtung verschließt, die an der Fertigspritze angebrachte Nadelschutzkappe mitabgezogen, d. h. beim Entfernen der Kappe von der Fertigspritze entfernt wird. Die Nadelschutzkappe verbleibt dabei in der Kappe. Hierzu weist die Kappe Eingriffsglieder auf, die beim Abziehen der Kappe in Eingriff mit der Nadelschutzkappe gebracht werden. Die Eingriffsglieder sind elastisch federnd quer zur Längsachse der Injektionsvorrichtung bewegbar. Beim Fortsetzen der Abziehbewegung des Abziehelements nehmen die Eingriffsglieder die Nadelschutzkappe mit, wodurch diese von der Fertigspritze abgezogen wird. Um ein sicheres Abziehen der Nadelschutzkappe durch das Entfernen der Kappe zu gewährleisten, ist es aus dem Stand der Technik bekannt, dass die mit der Kappe verbundenen Eingriffsglieder in Eingriff mit der Nadelschutzkappe gelangen.

Es ist eine Aufgabe der Erfindung, eine Injektionsvorrichtung und ein Verfahren zur Montage und/oder zum Vorbereiten einer Injektionsvorrichtung für die Verabreichung eines Produkts anzugeben, die oder das ein einfaches Einsetzen des Produktbehälters in die Injektionsvorrichtung und/oder ein sicheres Entfernen der Nadelschutzkappe von dem Produktbehälter erlaubt, ohne die Sterilität der Injektionsnadel und des Medikaments ungewollt zu gefährden.

Die Aufgabe wird mit der Injektionsvorrichtung nach Anspruch 1 und dem Verfahren nach Anspruch 15 gelöst. Vorteilhafte Weiterbildungen ergeben sich aus den abhängigen Ansprüchen, der Beschreibung und den Figuren.

Die Erfindung geht von einer Vorrichtung zur Verabreichung eines Produkts, nämlich von einer Injektionsvorrichtung mit einer Längsachse (L) aus. Die Injektionsvorrichtung kann als so genannter Autoinjektor ausgestaltet sein, der einen Mechanismus aufweist, der ein automatisches Ausschütten des Produkts, wie z. B. durch einen Energiespeicher, insbesondere eine Feder, und optional ein automatisches Einstechen und/oder Zurückziehen der Injektionsnadel bewirkt. Bei einem Autoinjektor wird die Kraft zum Ausschütten des Produkts durch den Energiespeicher, wie z.B. die Feder bereitgestellt. Die Injektionsvorrichtung kann alternativ als manuelle Injektionsvorrichtung ausgestaltet sein, d. h., dass die Kraft für die Ausschüttung des Produkts durch Muskelkraft, wie z. B. durch den Benutzer selbst erfolgt. Die Injektionsvorrichtung - egal ob es sich um einen Autoinjektor oder eine manuelle Injektionsvorrichtung handelt - kann eine Nadelschutzhülse aufweisen, die nach erfolgter Injektion distal über das distale Ende der Injektionsnadel steht oder relativ zu dem Gehäuse in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse beispielsweise auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors als Sichtschutz dient.

Die Injektionsvorrichtung weist einen Produktbehälter mit einer Injektionsnadel auf, wie z. B. eine aus dem Stand der Technik bekannte Fertigspritze oder allgemein Spritze. Der Produktbehälter kann einen z. B. hohlzylindrischen Produktbehälterabschnitt aufweisen, der einen Kolben verschiebbar lagert. Der Kolben kann mit dem Innenumfang des Produktbehälterabschnitts einen Dichtspalt bilden und so eine sterile Barriere bilden. Der Kolben kann z. B. mittels einer Kolbenstange der Injektionsvorrichtung in die distale Richtung verschoben werden, um über die Injektionsnadel Produkt aus dem Produktbehälter abzugeben. Die Injektionsnadel kann vorzugsweise unlösbar an dem Produktbehälter gebildet sein. Zum Beispiel kann der Produktbehälter einen Halteabschnitt, insbesondere einen Nadelhalteabschnitt, aufweisen, der distal des Produktbehälterabschnitts angeordnet ist und mit der Injektionsnadel unlösbar verbunden ist, und so zum Beispiel einen proximalen Teil der Injektionsnadel umgibt. Die Injektionsnadel kann somit von dem Halteabschnitt in die distale Richtung abragen. Der Halteabschnitt kann beispielsweise einen geringeren Außendurchmesser als der Produktbehälterabschnitt aufweisen. Der Produktbehälterabschnitt kann sich an seinem distalen Ende zu dem Halteabschnitt hin verjüngen.

Der hierin verwendete Begriff "distal" bezieht sich auf die Richtung, in die die Spitze der Injektionsnadel zeigt. Der hierin verwendete Begriff "proximal" bezieht sich auf die Richtung, die der distalen Richtung entgegengesetzt ist.

Ferner umfasst der hierin verwendete Begriff "entlang der Längsachse (L)" sowohl den Begriff "parallel zu der Längsachse (L)" wie auch den Begriff "ungefähr parallel zu der Längsachse (L)".

An dem Produktbehälter, beispielsweise an dem Halteabschnitt, ist eine Nadelschutzkappe, wie z. B. ein aus dem Stand der Technik bekanntes soft needle shield (SNS) oder rigid needle shield (RNS), befestigt, insbesondere lösbar befestigt. Die Nadelschutzkappe kann z. B. reib- oder formschlüssig oder kombiniert reib- und formschlüssig auf dem Halteabschnitt befestigt sein. Die Nadelschutzkappe umschließt die Injektionsnadel und dichtet sie in Bezug auf die Umgebung steril ab. Ein soft needle shield (SNS) umfasst oder besteht aus einem Elastomer, beispielsweise auf Gummi- oder Kautschukbasis gebildeten Teil, welches die Nadel umgibt. Das soft needle shield (SNS) weist an seinem Außenumfang eine weiche, wie z. B. aus einem gummi- oder kautschukartigen Material gebildete Oberfläche auf. Ein rigid needle shield (RNS) weist zumeist mehrere Teile auf, insbesondere ein elastomeres kappenförmiges inneres Teil und ein aus einem festen, d. h. nicht-elastomeren Kunststoff hergestelltes hülsenförmiges oder kappenförmiges äußeres Teil, welches das elastomere Teil aufnimmt und damit im Wesentlichen unlösbar verbunden ist. Das äußere hülsen- oder kappenförmige Teil umgibt das innere kappenförmige Teil und ist mit der inneren Kappe beispielsweise unlösbar verbunden, so dass die äußere und innere Kappe eine Einheit bilden. Das innere Teil kann aus einem härteren Kunststoff gebildet als das innere Teil sein. Das äußere Teil kann beispielsweise aus Polyethylen, Polystyrol, Polypropylen oder einem anderen geeigneten Kunststoff sein. Das innere Teil kann beispielsweise aus Gummi oder Kautschuk oder einem anderen geeigneten Material gebildet sein.

An dem distalen Ende der Injektionsvorrichtung oder eines Gehäuses, wie z. B. eines Aufnahmegehäuses der Injektionsvorrichtung kann eine Kappe, die auch als Verschlusskappe oder Abziehkappe bezeichnet werden oder ausgestaltet sein kann, befestigt sein und das distale Ende des Gehäuses oder des Aufnahmegehäuses verschließen. Die Injektionsvorrichtung kann ein Gehäuse, wie z.B. ein Aufnahmegehäuse der Injektionsvorrichtung zur Aufnahme des Produktbehälters umfassen, wobei der Produktbehälter eine fest verbundene Injektionsnadel aufweist und wobei an dem Produktbehälter die Nadelschutzkappe lösbar angeordnet ist. Die Nadelschutzkappe umschliesst die Injektionsnadel und dichtet die Injektionsnadel gegenüber der Umgebung steril ab. Die Kappe kann z. B. mit dem Gehäuse oder Aufnahmegehäuse reib- und/oder formschlüssig verbunden sein, wie z. B. verschnappt sein. Die Kappe kann z. B. während des Entfernens von der Injektionsvorrichtung oder dem Gehäuse mit einer Axialbewegung oder einer kombinierten Axial-Dreh-Bewegung von der Injektionsvorrichtung, wie z. B. dem Gehäuse oder Aufnahmegehäuse, abnehmbar sein.

Die Injektionsvorrichtung kann ferner einen Produktbehälterhalter umfassen, welcher mit dem Gehäuse der Injektionsvorrichtung fest, insbesondere axial- und drehfest verbunden ist. Der Produktbehälterhalter kann zur Aufnahme des Produktbehälters dienen, wobei in dem Produktbehälterhalter der Produktbehälter fest, insbesondere axial- und vorzugsweise drehfest gehalten werden kann. Alternativ können das Gehäuse und der Produktbehälterhalter einteilig ausgebildet sein. Alternativ kann der Produktbehälterhalter relativ zu dem Gehäuse axial bewegbar und/oder drehbar angeordnet sein.

Die Kappe, welche lösbar an dem distalen Ende des Gehäuses der Injektionsvorrichtung vorgesehen ist, umfasst ein oder mehrere Eingriffselemente, um beim Entfernen der Kappe von der Injektionsvorrichtung das Entfernen der Nadelschutzkappe von dem Produktbehälter zu bewirken. Die Kappe, welche mit dem Eingriffselement gekoppelt ist, kann über das Eingriffselement derart mit der Nadelschutzkappe verbindbar sein, dass das Entfernen der Kappe von der Injektionsvorrichtung das Entfernen der Nadelschutzkappe von dem Produktbehälter bewirkt. Insbesondere kann zumindest ein Teil der Bewegung oder die gesamte Bewegung der Kappe in die distale Richtung auf das Eingriffselement übertragen werden, d. h., dass das Eingriffselement von der Kappe mitgenommen wird, so dass das Eingriffselement die Nadelschutzkappe von dem Produktbehälter, insbesondere dem Halteabschnitt, abzieht.

Das Eingriffselement ist derart verformbar, dass das Eingriffselement von einer beabstandeten Position, in welcher das Eingriffselement von der Nadelschutzkappe radial beabstandet ist, in eine Eingriffsposition, in welcher das Eingriffselement in Eingriff mit der Nadelschutzkappe ist, gelangbar ist, wobei das Eingriffselement beim Entfernen der Kappe verformt wird. Ferner ist das Eingriffselement derart ausgebildet, dass bei der Montage der Injektionsvorrichtung, insbesondere beim Einfügen des Produktbehälters in das Gehäuse oder in den Produktbehälterhalter, keine oder sehr wenige Kräfte, insbesondere keine oder sehr wenige von dem Eingriffselement ausgeübte Kräfte auf die Nadelschutzkappe wirken. Hierdurch wird verhindert, dass die Nadelschutzkappe bereits während des Einfügens des Produktbehälters relativ zu dem Produktbehälter bewegt wird. Dadurch wird die Gefahr verringert, dass die Sterilität der Injektionsnadel und des Medikaments leidet. Durch diese Anordnung kann ferner erzielt werden, dass während der Lagerung des Injektionsgeräts keine oder sehr wenige von dem Eingriffselement ausgeübte Kräfte auf die Nadelschutzkappe wirken.

Das Eingriffselement kann z. B. im Auslieferungszustand der Injektionsvorrichtung in Bezug auf die Nadelschutzkappe in der beabstandeten Position sein. In der Eingriffsposition ist das Eingriffselement in Bezug auf die Nadelschutzkappe derart angeordnet, dass eine Bewegung der Kappe in die distale Richtung eine Mitnahme der Nadelschutzkappe bewirkt und somit die Nadelschutzkappe von dem Produktbehälter entfernt wird. In der Eingriffsposition des Eingriffselements greift das Eingriffselement an oder in die Nadelschutzkappe. Das Eingriffselement kann an oder in eine Mantelfläche oder an oder in eine Kante oder an oder in eine distalen Stirnfläche oder an oder in eine proximale Stirnfläche der Nadelschutzkappe greifen. Besonders bevorzugt kann das Eingriffselement hakenförmig ausgebildet sein. Das hackenförmige Eingriffselement kann einen kurzen und einen langen Schenkel aufweisen. Alternativ kann das Eingriffselement eine andere Ausgestaltung aufweisen, wobei in der beabstandeten Position des Eingriffselements das Eingriffselement von der Nadelschutzkappe radial beabstandet ist und in der Eingriffsposition des Eingriffselements das Eingriffselement in Eingriff mit der Nadelschutzkappe ist, wobei das Eingriffselement beim Entfernen der Kappe verformt wird.

In der beabstandeten Position des Eingriffselements kann das Eingriffselement unverformt, verformt oder radial nach aussen verformt sein. In der Eingriffsposition des Eingriffselements kann das Eingriffselement unverformt, verformt oder radial nach innen verformt sein. Das Eingriffselement ist plastisch verformbar.

Als eine plastische Verformung wird eine irreversible Verformung bezeichnet. Die Verformung eines Materials ist plastisch, wenn das Material nicht wieder von allein seine ursprüngliche Form annimmt. Nach Einwirkung von einer Kraft oder Belastung auf das Material behält das Material seine Form bei.

Das Eingriffselement ist vorzugsweise aus Metall, insbesondere aus Stahl, besonders bevorzugt aus rostfreiem Stahl oder Federstahl gebildet. Das Eingriffselement ist aus einem Material gebildet, welches eine Biegefestigkeit aufweist, welche eine plastische Verformung zulässt. Das Eingriffselement ist derart ausgebildet, dass es in der beabstandeten Position des Eingriffselements plastisch verformt und in der Eingriffsposition plastisch unverformt ausgebildet ist oder, dass es in der beabstandeten Position des Eingriffselements plastisch unverformt und in der Eingriffsposition plastisch verformt ausgebildet ist.

Besonders bevorzugt kann das Eingriffselement in der beabstandeten Position des Eingriffselements radial nach aussen plastisch verformt oder alternativ plastisch unverformt ausgebildet sein. Besonders bevorzugt kann das Eingriffselement in der Eingriffsposition des Eingriffselements plastisch unverformt oder alternativ radial nach innen plastisch verformt ausgebildet sein. Besonders bevorzugt kann in der beabstandeten Position des Eingriffselements der lange Schenkel des hakenförmigen Eingriffselements radial nach aussen plastisch verformt sein oder alternativ plastisch unverformt sein und der kurze Schenkel des hakenförmigen Eingriffselements radial nach innen ragen. In der Eingriffsposition des Eingriffselements kann besonders bevorzugt der lange Schenkel des hakenförmigen Eingriffselements plastisch unverformt ausgebildet sein oder alternativ plastisch verformt, insbesondere radial nach innen verformt sein und der kurze Schenkel des hakenförmigen Eingriffselements radial nach innen ragen, wobei der kurze Schenkel des Eingriffselements mit der Manteloberfläche der Nadelschutzkappe in Eingriff, insbesondere immer in Eingriff ist. Die Mantelfläche der Nadelschutzkappe kann eine oder mehrere Öffnungen oder ein oder mehrere Befestigungsmittel aufweisen, in welche das Eingriffselement in der Eingriffsposition des Eingriffselements eingreifen oder einbohren kann. Alternativ weist die Nadelschutzkappe keine Öffnung oder kein Befestigungsmittel auf, wobei das Eingriffselement in der Eingriffsposition des Eingriffselements in die Mantelfläche der Nadelschutzkappe eingreifen oder einbohren kann.

Das hakenförmige Eingriffselement kann einen langen und einen kurzen Schenkel aufweisen, wobei der lange und der kurze Schenkel miteinander verbunden sind. Das Eingriffselement kann aus einem Stanzbiegeteil gebildet sein. Das Stanzbiegeteil ist plastisch verformbar. Das Stanzbiegeteil ist vorzugsweise aus Metall, insbesondere aus Stahl, besonders bevorzugt aus rostfreiem Stahl oder Federstahl gebildet. Das Stanzbiegeteil ist aus einem Material gebildet, welches eine Biegefestigkeit aufweist, welche eine plastische Verformung zulässt. Der lange Schenkel des Eingriffselements kann unter einem Winkel quer zur Längsachse (L) verformbar sein. Der lange Schenkel des Eingriffselements kann unter einem Winkel quer zur Längsachse (L), insbesondere unter einem Winkel von weniger als 90° quer zur Längsachse (L) radial nach innen oder radial nach aussen verformbar sein. Der lange Schenkel erstreckt sich entlang der Längsachse (L), wobei der lange Schenkel radial nach innen oder radial nach aussen plastisch verformbar sein kann. Der kurze Schenkel des Eingriffselements ragt radial nach innen. Der kurze Schenkel des Eingriffselements ist vorzugsweise zahn- oder dreieckförmig oder spitzwinklig ausgebildet. Die Seiten des zahn- oder dreieckförmigen oder spitzwinkligen kurzen Schenkels können gerade oder gekrümmt ausgebildet sein. Vorzugsweise weist der kurze Schenkel zwei gekrümmte und eine gerade Seite auf. Eine Spitze des zahn- oder dreieckförmigen oder spitzwinkligen kurzen Schenkels des Eingriffselements kann vorzugsweise radial nach innen ragen. Die Spitze des zahn- oder dreieckförmigen oder spitzwinkligen kurzen Schenkels ist vorzugsweise gegenüber der geraden Seite des zahn- oder dreieckförmigen oder spitzwinkligen kurzen Schenkels angeordnet. Die Spitze des kurzen Schenkels kann krallenförmig ausgebildet sein. Die Spitze des kurzen Schenkels des Eingriffselements kann in der Eingriffsposition, in welcher das Eingriffselement mit der Nadelschutzkappe in Eingriff ist, in Eingriff mit der Nadelschutzkappe sein, insbesondere immer in Eingriff mit der Nadelschutzkappe sein. Der kurze Schenkel des Eingriffselements ist derart ausgebildet, dass der kurze Schenkel in Eingriff mit der Nadelschutzkappe gelangen kann oder in Eingriff mit der Nadelschutzkappe sein kann.

In einer Ausführungsform erstreckt sich der lange Schenkel entlang der Längsachse (L) und der lange und der kurze Schenkel des Eingriffselements können derart miteinander plastisch verformt verbunden sein, dass sich der kurze Schenkel von dem langen Schenkel unter einem Winkel, insbesondere unter einem Winkel von weniger als 90° quer zur Längsachse (L) radial nach innen erstreckt. Besonders bevorzugt können der lange und der kurze Schenkel des Eingriffselements derart miteinander verbunden sein, dass die gerade Seite des zahn- oder dreieckförmigen oder spitzwinkligen kurzen Schenkel unter einem Winkel, insbesondere unter einem Winkel von 90° quer zur Längsachse (L) mit dem langen Schenkel verbunden ist und die Spitze des kurzen Schenkels des Eingriffselements radial nach innen ragt. Beim Entfernen der Kappe, wobei das Eingriffselement in der Eingriffsposition ist, wirkt die Zugkraft quer zur geraden Seite des zahn- oder dreieckförmigen oder spitzwinkligen kurzen Schenkels des Eingriffselements, wobei die gerade Seite des zahn- oder dreieckförmigen oder spitzwinkligen kurzen Schenkels unter einem Winkel, insbesondere unter einem Winkel von 90° quer zur Längsachse (L) mit dem langen Schenkel verbunden ist. Die Dicke des zahn- oder dreieckförmigen oder spitzwinkligen kurzen Schenkels, insbesondere die Dicke des Stanzbiegeteils oder des Metalls für den zahn- oder dreieckförmigen oder spitzwinkligen kurzen Schenkel kann derart angepasst sein oder werden, dass die Kappe von der Injektionsvorrichtung sicher entfernt werden kann.

In einer alternativen Ausführungsform erstreckt sich der lange Schenkel entlang der Längsachse (L) und der lange und der kurze Schenkel des Eingriffselements können derart miteinander plastisch verformt verbunden sein, dass sich der kurze Schenkel von dem langen Schenkel unter einem Winkel, insbesondere unter einem Winkel zur Längsachse (L) entlang der Längsachse (L) radial nach innen erstreckt. Besonders bevorzugt können der lange und der kurze Schenkel des Eingriffselements derart miteinander verbunden sein, dass die gerade Seite des zahn- oder dreieckförmigen oder spitzwinkligen kurzen Schenkels entlang der Längsachse (L) mit dem langen Schenkel verbunden ist und die Spitze des kurzen Schenkels des Eingriffselements radial nach innen ragt. Beim Entfernen der Kappe, wobei das Eingriffselement in der Eingriffsposition ist, wirkt die Zugkraft entlang der geraden Seite des zahn- oder dreieckförmigen oder spitzwinkligen kurzen Schenkels des Eingriffselements, wobei die gerade Seite des zahn- oder dreieckförmigen oder spitzwinkligen kurzen Schenkels entlang der Längsachse (L) mit dem langen Schenkel verbunden ist. Die Länge der geraden Seite des zahn- oder dreieckförmigen oder spitzwinkligen kurzen Schenkels, insbesondere die Länge der geraden Seite des zahn- oder dreieckförmigen oder spitzwinkligen kurzen Schenkels, welche mit dem langen Schenkel verbunden ist, kann derart angepasst sein oder werden, dass die Kappe sicher von der Injektionsvorrichtung abgezogen werden kann.

Ferner können an dem langen Schenkel mehrere kurze Schenkel vorgesehen sein. Die mehreren kurzen Schenkel können um die Längsachse (L) in Umfangsrichtung, quer zur Längsachse (L) und/oder entlang der Längsachse (L) angeordnet sein. Die mehreren kurzen Schenkel können verschieden ausgestaltet sein. Vorzugsweise sind die kurzen Schenkel zahn- oder dreieckförmig oder spitzwinkligen ausgebildet. Der Abstand zwischen der geraden Seite und der Spitze des zahn- oder dreieckförmigen oder spitzwinkligen kurzen Schenkels kann verschieden sein. Die mehreren kurzen Schenkel bewirken ein sicheres Eingreifen oder einen sicheren Eingriff in die Nadelschutzkappe, wobei die Nadelschutzkappe von der Injektionsvorrichtung abgezogen werden kann. Durch die verschiedenen Abstände zwischen der geraden Seite und der Spitze des zahn- oder dreieckförmigen oder spitzwinkligen kurzen Schenkels können die Schenkel versetzt zur Längsachse (L) in die Nadelschutzkappe eingreifen, wobei die Nadelschutzkappe sicher von der Injektionsvorrichtung abgezogen werden kann.

Der lange Schenkel des Eingriffselements ist derart verformbar, insbesondere der lange Schenkel des Eingriffselements ist unter einem Winkel von weniger als 90° quer zur Längsachse (L) derart plastisch verformbar, dass der kurze Schenkel des Eingriffselements von einer beabstandeten Position, in welcher der kurze Schenkel des Eingriffselements von der Nadelschutzkappe radial beabstandet ist, in eine Eingriffsposition, in welcher der kurze Schenkel des Eingriffselements in Eingriff mit der Nadelschutzkappe ist, gelangbar ist, wobei der lange Schenkel des Eingriffselements beim Entfernen der Kappe plastisch verformt wird. Der lange Schenkel des Eingriffselements kann radial nach innen oder radial nach aussen plastisch verformt werden.

Alternativ oder zusätzlich kann das Eingriffselement, insbesondere der kurze Schenkel des Eingriffselements ein Befestigungselement aufweisen, welches in der Eingriffsposition des Eingriffselements eine feste, insbesondere eine axial- und radialfeste Verbindung mit der Nadelschutzkappe eingehen kann. Das Befestigungselement des Eingriffselements kann mit der Mantelfläche oder mit einer Kante der Nadelschutzkappe eine feste Verbindung bilden. An der Mantelfläche oder an der Kante der Nadelschutzkappe können eine oder mehrere Öffnungen oder ein oder mehrere Befestigungsmittel vorgesehen sein, wobei das Befestigungselement des Eingriffselements eine feste Verbindung mit der Öffnung oder dem Befestigungsmittel der Nadelschutzkappe eingehen kann. Alternativ weist die Nadelschutzkappe keine Öffnung oder kein Befestigungsmittel auf, wobei das Befestigungselement des Eingriffselements in die Mantelfläche oder in die Kante der Nadelschutzkappe eingreifen oder einbohren kann.

Ferner kann die Kappe eine Hülse aufweisen, wobei die Hülse das Eingriffselement zumindest teilweise umfassen kann. Die Hülse kann vorzugsweise eine Innenhülse und eine Aussenhülse aufweisen. Das Eingriffselement kann mit der Hülse axial bewegbar oder vorzugweise axialfest verbunden sein. Vorzugsweise kann das Eingriffselement mit der Innenhülse axial bewegbar oder vorzugsweise axialfest verbunden sein. Die Hülse kann vorzugsweise aus Kunststoff gebildet sein. Alternativ kann die Hülse aus Metall gebildet sein. Die Hülse kann z.B. mit dem Gehäuse der Injektionsvorrichtung reib- und/oder formschlüssig verbunden sein, wie z.B. verschnappt sein. Dazu kann z.B. an der Aussenhülse der Hülse ein Eingriffsglied vorgesehen sein, welches in ein an dem Gehäuse angebrachtes Gegeneingriffsglied lösbar in Eingriff sein kann. Die Hülse kann z.B. während des Entfernens der Kappe von der Injektionsvorrichtung oder dem Gehäuse mit einer Axialbewegung oder einer kombinierten Axial-Dreh-Bewegung von der Injektionsvorrichtung, wie z.B. dem Gehäuse abnehmbar sein.

Die Kappe kann ferner ein hülsenförmiges oder zylinderförmiges Entfernerelement aufweisen, wobei an dem Entfernerelement das Eingriffselement vorgesehen sein kann. Besonders bevorzugt kann der lange Schenkel des Eingriffselements mit dem Entfernerelement verbunden sein. Das Entfernerelement kann hülsenförmig oder zylinderförmig ausgebildet sein, wobei an einem proximalen Ende des Entfernerelements das Eingriffselement, insbesondere der lange Schenkel des Eingriffselements angebracht sein kann. Alternativ kann das Eingriffselement in und/oder an einer Mantelfläche des Entfernerelements, insbesondere in und/oder an einer Mantelfläche des hülsenförmigen oder zylinderförmigen Entfernerelements vorgesehen sein. Der lange Schenkel des Eingriffselements kann in und/oder an der Mantelfläche des hülsenförmigen oder zylinderförmigen Entfernerelements angebracht sein. Das Entfernerelement und das Eingriffselement sind vorzugsweise axial- und drehfest miteinander verbunden sein. Das Entfernerelement und das Eingriffselement können ein- oder zweistückig ausgebildet sein. Das Entfernerelement und das Eingriffselement können vorzugsweise aus dem gleichen Material gebildet sein. Besonders bevorzugt sind das Entfernerelement und das Eingriffselement aus einem Stanzbiegeteil gebildet. Das Stanzbiegeteil ist plastisch verformbar. Das Stanzbiegeteil ist vorzugsweise aus Metall, insbesondere aus Stahl, besonders bevorzugt aus rostfreiem Stahl oder Federstahl gebildet. Das Stanzbiegeteil ist aus einem Material gebildet, welches eine Biegefestigkeit aufweist, welche eine plastische Verformung zulässt. Das Stanzbiegeteil kann in eine Hülsenform oder in eine Zylinderform gebogen werden. Alternativ können das Entfernerelement und das Eingriffselement aus verschiedenen Materialien gebildet sein. Die Hülse kann das beispielsweise hülsenförmige oder zylinderförmige Entfernerelement umgeben, vorzugsweise über seinen Umfang einfassen. Das Entfernerelement ist vorzugsweise ein von der Hülse separates Element, welches jedoch mit der Hülse verbunden, wie z. B. entlang der Längsachse (L) verschiebbar oder unverschiebbar verbunden sein kann. Das Entfernerelement kann vorzugweise ein oder mehrere Vorsprünge aufweisen, welche in an der Hülse vorgesehene Ausnehmungen eingerastet sind, um eine axialfeste Verbindung zwischen der Hülse und dem Entfernerelement zu bilden. Vorzugsweise kann das Entfernerelement mit der Innenhülse der Hülse verschiebbar oder unverschiebbar verbunden sein, wobei die Ausnehmungen an der Innenhülse vorgesehen sind.

Ferner kann an dem Gehäuse oder an einem Gehäuse fest verbundenen Teil ein oder mehrere Sperrelemente vorgesehen sein, wobei in der Eingriffsposition das oder die Sperrelemente das Eingriffselement in Eingriff mit der Nadelschutzkappe hält oder bringt. Das Sperrelement kann eine erste und/oder eine zweite schräge Fläche, insbesondere eine erste und/oder eine zweite nach innen ragende schräge Fläche umfassen. Die erste und/oder die zweite schräge Fläche des Sperrelements können eine Neigung aufweisen. Die erste und die zweite schräge Fläche können zueinander geneigt sein. Das Eingriffselement der Kappe ist derart mit dem Sperrelement des Gehäuses gekoppelt, dass während dem Entfernen der Kappe von der Injektionsvorrichtung das Eingriffselement relativ zu der Nadelschutzkappe bewegbar ist oder bewegt wird und bei dieser Bewegung, insbesondere einer Axialbewegung oder einer kombinierten Axial-Dreh-Bewegung mittels dem Sperrelement des Gehäuses derart verformbar ist oder verformt wird, dass das Eingriffselement, insbesondere der kurze Schenkel des Eingriffselements in Eingriff mit der Nadelschutzkappe gelangbar ist oder gelangt. In der Eingriffsposition des Eingriffselements ist das Eingriffselement mit der Nadelschutzkappe axialfest verbunden, wobei die Nadelschutzkappe von dem Eingriffselement der Kappe bei der Fortführung der Axialbewegung oder der kombinierten Axial-Drehbewegung der Kappe mitgenommen wird. Mit anderen Worten umfasst der Hub, den die Kappe beim Entfernen von der Injektionsvorrichtung relativ dem Gehäuse entlang der Längsachse (L) in die distale Richtung ausführt, einen ersten Teilhub, während dem die Kappe relativ zu der Nadelschutzkappe bewegbar ist oder bewegt wird, und einen zweiten Teilhub, während dem die Nadelschutzkappe die Bewegung der Kappe mitmacht oder von der Kappe mitgenommen wird.

Die Erfindung betrifft auch ein Verfahren zum Montieren einer Injektionsvorrichtung und/oder zum Vorbereiten einer Injektionsvorrichtung für die Verabreichung eines Produkts. Bei der Injektionsvorrichtung kann es sich z. B. um die hierin beschriebene Injektionsvorrichtung handeln.

Das Verfahren umfasst den Schritt des Bereitstellens eines Gehäuses oder eines Aufnahmegehäuses, welches z. B. Teil eines Gehäuses der Injektionsvorrichtung sein kann, zur Aufnahme eines Produktbehälters. Das Gehäuse beziehungsweise das Aufnahmegehäuse kann z. B. hülsenförmig und/oder länglich ausgestaltet sein.

Das Verfahren umfasst ferner den Schritt des Bereitstellens einer Kappe, welche an einem distalen Ende des Gehäuses lösbar angebracht werden kann. Ferner umfasst das Verfahren den Schritt Anbringen der Kappe an dem distalen Ende des Gehäuses. Die Kappe kann z.B. mit dem Gehäuse verschnappt werden. Die Kappe umfasst ein oder mehrere Eingriffselemente, um beim Entfernen der Kappe von der Injektionsvorrichtung das Entfernen der Nadelschutzkappe von dem Produktbehälter zu bewirken. Das Eingriffselement kann hakenförmig ausgebildet sein. Das hakenförmige Eingriffselement kann einen langen und einen kurzen Schenkel aufweisen, wobei der lange und der kurze Schenkel miteinander verbunden sind.

Die Kappe kann ferner ein Entfernerelement umfassen, wobei an dem Entfernerelement das Eingriffselement angeordnet ist. Besonders bevorzugt kann der lange Schenkel des Eingriffselements mit dem Entfernerelement verbunden sein. Das Entfernerelement und das Eingriffselement können ein- oder zweistückig ausgebildet sein. Ferner kann die Kappe eine Hülse umfassen, wobei die Hülse das beispielsweise hülsenförmige oder zylinderförmige Entfernerelement umgeben kann, vorzugweise über seinen Umfang einfassen kann. Das Entfernerelement ist vorzugsweise ein von der Hülse separates Element, welches jedoch mit der Hülse verbunden, wie z. B. entlang der Längsachse (L) verschiebbar oder vorzugsweise unverschiebbar verbunden sein kann. Das Eingriffselement und vorzugsweise das Entfernerelement sind vorzugsweise aus einem anderen Material gebildet als die Hülse. Die Hülse ist vorzugweise aus Kunststoff gebildet. Das Eingriffselement und das Entfernerelement sind vorzugsweise aus dem gleichen Material gebildet.

Das Verfahren umfasst ferner das Bereitstellen des Produktbehälters, der eine fest verbundene Injektionsnadel aufweist, wobei an dem Produktbehälter eine Nadelschutzkappe lösbar angeordnet ist, welche die Injektionsnadel umschliesst und gegenüber der Umgebung steril abdichtet. Der Produktbehälter kann z.B. eine aus dem Stand der Technik bekannte Fertigspritze oder allgemein Spritze sein. Der Produktbehälter kann einen z. B. hohlzylindrischen Produktbehälterabschnitt umfassen, wobei in dem Produktbehälter ein Kolben verschiebbar angeordnet ist. Der Kolben dient dazu, um z. B. mittels einer Kolbenstange der Injektionsvorrichtung über die Injektionsnadel Produkt aus dem Produktbehälter abzugeben. An dem Produktbehälter kann vorzugsweise die Injektionsnadel unlösbar gebildet sein. Ferner kann der Produktbehälter einen Halteabschnitt, insbesondere einen Nadelhalteabschnitt, umfassen, der distal des Produktbehälterabschnitts angeordnet ist und mit der Injektionsnadel unlösbar verbunden ist. Der Nadelhaltabschnitt des Produktbehälters kann einen proximalen Teil der Injektionsnadel umgeben. Die Injektionsnadel kann somit von dem Halteabschnitt in die distale Richtung abragen. Der Halteabschnitt kann einen geringeren Außendurchmesser als der Produktbehälterabschnitt aufweisen. Der Produktbehälterabschnitt kann sich an seinem distalen Ende zu dem Halteabschnitt hin verjüngen. An dem Produktbehälter, insbesondere an dem Halteabschnitt des Produktbehälters ist die Nadelschutzkappe angeordnet, welche die Injektionsnadel umschließt und gegenüber der Umgebung vorzugsweise steril abdichtet.

Das Verfahren umfasst ferner das Verschieben oder Einsetzen des Produktbehälters mit der lösbar verbundenen Nadelschutzkappe in das Gehäuse entlang einer Längsachse (L) in eine distale Richtung, wobei das Eingriffselement, insbesondere der lange Schenkel des Eingriffselements derart verformbar oder verformt oder vorverformt ist, dass beim Verschieben oder Einsetzen des Produktbehälters relativ zu dem Gehäuse in die distale Richtung die Nadelschutzkappe von dem Eingriffselement radial beabstandet ist. Dadurch wird erreicht, dass beim Einfügen des Produktbehälters keine oder sehr wenige Kräfte auf die Nadelschutzkappe ausgeübt werden. Hierdurch wird verhindert, dass die Nadelschutzkappe bereits während des Einfügens des Produktbehälters relativ zu dem Produktbehälter bewegt wird. Dadurch wird die Gefahr verringert, dass die Sterilität der Injektionsnadel und des Medikaments leidet.

Das Eingriffselement, insbesondere der lange Schenkel des Eingriffselements ist vorzugsweise derart verformbar, dass während dem Entfernen der Kappe von der Injektionsvorrichtung das Eingriffselement in Eingriff mit der Nadelschutzkappe gebracht werden kann.

In der beabstandeten Position des Eingriffselements kann das Eingriffselement, insbesondere der lange Schenkel des Eingriffselements unverformt, verformt oder radial nach aussen verformt sein. In der Eingriffsposition des Eingriffselements kann das Eingriffselement, insbesondere der lange Schenkel des Eingriffselements unverformt, verformt oder radial nach innen verformt sein. Das Eingriffselement, insbesondere der lange Schenkel des Eingriffselements ist plastisch verformbar. Das Eingriffselement, insbesondere der lange Schenkel des Eingriffslements ist vorzugsweise aus Metall, insbesondere aus Stahl, besonders bevorzugt aus rostfreiem Stahl oder Federstahl gebildet. Das Eingriffselement, insbesondere der lange Schenkel des Eingriffselements ist aus einem Material gebildet, welches eine Biegefestigkeit aufweist, welche eine plastische Verformung zulässt.

Alternativ kann das Eingriffselement eine andere Ausgestaltung als eine hakenförmige Gestalt aufweisen, wobei in der beabstandeten Position des Eingriffselements das Eingriffselement von der Nadelschutzkappe radial beabstandet ist und in der Eingriffsposition des Eingriffselements das Eingriffselement in Eingriff mit der Nadelschutzkappe ist, wobei das Eingriffselement beim Entfernen der Kappe verformt wird.

Besonders bevorzugt ist das Eingriffselement, insbesondere der lange Schenkel des Eingriffselements derart ausgebildet, dass es, insbesondere er in der beabstandeten Position des Eingriffselements plastisch verformt und in der Eingriffsposition plastisch unverformt ist oder, dass es, insbesondere er in der beabstandeten Position des Eingriffselements plastisch unverformt und in der Eingriffsposition plastisch verformt ist.

Besonders bevorzugt kann das Eingriffselement in der beabstandeten Position des Eingriffselements radial nach aussen plastisch verformt oder alternativ plastisch unverformt ausgebildet sein. Besonders bevorzugt kann das Eingriffselement in der Eingriffsposition des Eingriffselements plastisch unverformt oder alternativ radial nach innen plastisch verformt ausgebildet sein. Besonders bevorzugt kann in der beabstandeten Position des Eingriffselements der lange Schenkel des hakenförmigen Eingriffselements radial nach aussen plastisch verformt sein oder alternativ plastisch unverformt sein und der kurze Schenkel des hakenförmigen Eingriffselements radial nach innen ragen. In der Eingriffsposition des Eingriffselements kann besonders bevorzugt der lange Schenkel des hakenförmigen Eingriffselements plastisch unverformt ausgebildet sein oder alternativ plastisch verformt, insbesondere radial nach innen verformt sein und der kurze Schenkel des hakenförmigen Eingriffselements radial nach innen ragen, wobei der kurze Schenkel des Eingriffselements mit einer Manteloberfläche der Nadelschutzkappe in Eingriff ist, insbesondere immer in Eingriff ist.

Vor dem Verschieben oder Einsetzen des Produktbehälters relativ zu dem Gehäuse in die distale Richtung, insbesondere vor dem Verschieben oder Einsetzen des Produktbehälters in das Gehäuse der Injektionsvorrichtung wird das Eingriffselement verformt, vorzugsweise radial nach aussen verformt. Beispielsweise kann dabei der lange Schenkel des hakenförmigen Eingriffselements verformt werden, vorzugsweise radial nach aussen verformt werden, insbesondere radial nach aussen plastisch verformt werden, wobei der kurze Schenkel radial nach innen ragt. Der lange Schenkel des Eingriffselements kann unter einem Winkel, insbesondere unter einem Winkel von weniger als 90° quer zur Längsachse (L) plastisch verformt, insbesondere radial nach aussen plastisch verformt werden. Der kurze Schenkel ragt dabei radial nach innen. Für diese Verformung kann ein Montagewerkzeug verwendet werden, um das Eingriffselement in eine beabstandete Position, in welcher das Eingriffselement von der Nadelschutzkappe radial beabstandet ist, zu bringen. Das Montagewerkzeug kann als Montagedorn oder als Spreizdorn ausgebildet sein. Dabei kann das Eingriffselement plastisch verformt werden, wobei das Eingriffselement aus einem plastischen Material gebildet ist.

Um das hakenförmige Eingriffselement zu bilden, kann ein Stanzbiegeteil verwendet werden. Das Stanzbiegeteil ist plastisch verformbar. Aus einem Metall, welches vorzugsweise aus Stahl, besonders bevorzugt aus rostfreiem Stahl, insbesondere aus rostfreiem Stahl oder Federstahl gebildet ist, werden mindestens ein langer Schenkel und mindestens ein kurzer Schenkel des Eingriffselements gestanzt. Der lange und der kurze Schenkel des Eingriffselements sind miteinander verbunden. Der lange Schenkel erstreckt sich entlang der Längsachse (L). In einer Ausführungsform wird die Verbindung zwischen dem langen und dem kurzen Schenkel derart plastisch verformt, dass sich der kurze Schenkel von dem langen Schenkel unter einem Winkel, insbesondere unter einem Winkel von weniger als 90° Winkel quer zur Längsachse (L) radial nach innen erstreckt. In einer alternativen Ausführungsform wird die Verbindung zwischen dem langen und dem kurzen Schenkel derart verformt, insbesondere plastisch verformt, dass sich der kurze Schenkel von dem langen Schenkel unter einem Winkel, insbesondere unter einem Winkel zur Längsachse (L) entlang der Längsachse (L) radial nach innen erstreckt.

Ferner kann aus dem Metall mindestens ein langer Schenkel, wobei an dem langen Schenkel mehrere kurze Schenkel vorgesehen sind, gestanzt werden, um das Eingriffselement zu bilden.

Ferner kann der lange Schenkel unter einem Winkel, unter einem Winkel von weniger als 90° quer zur Längsachse (L) plastisch verformt werden, sodass das Eingriffselement in eine beabstandete Position und/oder in eine Eingriffsposition gelangt. Der lange Schenkel kann unter einem Winkel, insbesondere unter einem Winkel von weniger als 90° quer zur Längsachse (L) radial nach innen oder radial nach aussen plastisch verformt werden.

Das Stanzbiegeteil kann das Eingriffselement und das Entfernerelement umfassen. Aus dem Stanzbiegeteil kann das Eingriffselement und das Entfernerelement gestanzt werden, wobei nach dem Stanzen das Entfernerelement, insbesondere das Entfernerelement und/oder das Eingriffselement in eine Hülsenform oder Zylinderform geformt werden kann.

Das Eingriffselement, insbesondere der lange Schenkel des Eingriffselements der Kappe kann vor dem lösbaren Anbringen der Kappe auf das Gehäuse der Injektionsvorrichtung radial nach aussen plastisch verformt werden. Alternativ kann das Eingriffselement, insbesondere der lange Schenkel des Eingriffselements der Kappe radial nach aussen plastisch verformt werden, nachdem die Kappe lösbar an das Gehäuse der Injektionsvorrichtung angebracht worden ist. Alternativ kann beim Anbringen der Kappe auf das Gehäuse das Eingriffselement, insbesondere der lange Schenkel des Eingriffselements radial nach innen plastisch verformt werden.

Das Eingriffselement kann aufgrund des Materials, insbesondere der Biegefestigkeit des Materials plastisch verformt bleiben oder alternativ mittels Teil der Injektionsvorrichtung, insbesondere einem Teil des Gehäuses oder einem Produktbehälterhalter, wobei der Produktbehälter in dem Produktbehälterhalter aufnehmbar ist, in der plastischen Form gehalten werden.

An dem Gehäuse oder an einem Gehäuse fest verbundenen Teil oder an dem Produktbehälterhalter sind vorzugsweise ein oder mehrere Sperrelemente vorgesehen, welche das Eingriffselement in Eingriff mit der Nadelschutzkappe halten oder bringen. Das Sperrelement weist eine erste und/oder eine zweite schräge Fläche, insbesondere eine erste und/oder eine zweite nach innen ragende schräge Fläche auf. Die erste und/oder die zweite schräge Fläche des Sperrelements können eine Neigung aufweisen. Die erste und die zweite schräge Fläche können zueinander geneigt sein. Wenn das Eingriffselement in Eingriff mit der Nadelschutzkappe ist, ist das Eingriffselement in der Eingriffsposition. In der Eingriffsposition des Eingriffselements kann das Eingriffselement mit der Nadelschutzkappe eine feste, insbesondere axial- und radialfeste Verbindung bilden. Vorzugsweise verbindet sich das Eingriffselement mit einer Mantelfläche der Nadelschutzkappe, wobei das Eingriffselement in die Mantelfläche der Nadelschutzkappe eingreift oder eingebohrt ist. In der Eingriffsposition des Eingriffselements greift das Eingriffselement an oder in die Nadelschutzkappe. Das Eingriffselement, insbesondere der kurze Schenkel des Eingriffselements kann an oder in eine Mantelfläche oder an oder in eine Kante oder an oder in eine distalen Stirnfläche oder an oder in eine proximale Stirnfläche der Nadelschutzkappe greifen. Ferner kann die Mantelfläche der Nadelschutzkappe eine oder mehrere Öffnungen oder ein oder mehrere Befestigungsmittel umfassen, wobei sich das Eingriffselement in der Eingriffsposition des Eingriffselements in diese Öffnung oder in dieses Befestigungsmittel eingreifen oder einbohren kann. Alternativ weist die Nadelschutzkappe keine Öffnung oder kein Befestigungsmittel auf, wobei das Eingriffselement, insbesondere der kurze Schenkel des Eingriffselements in der Eingriffsposition des Eingriffselements sich in die Mantelfläche der Nadelschutzkappe eingreifen oder einbohren kann.

Das Eingriffselement, insbesondere der kurze Schenkel des Eingriffselements kann ferner ein Befestigungselement umfassen. Das Befestigungselement kann sich in der Eingriffsposition des Eingriffselements mit der Nadelschutzkappe fest, insbesondere axial- und/oder radialfest verbinden. Das Befestigungselement des Eingriffselements kann sich mit der Mantelfläche oder mit einer Kante der Nadelschutzkappe fest verbinden. An der Mantelfläche oder an der Kante der Nadelschutzkappe können eine oder mehrere Öffnungen oder ein oder mehrere Befestigungsmittel vorgesehen sein, wobei sich das Befestigungselement des Eingriffselements mit der Öffnung oder dem Befestigungsmittel der Nadelschutzkappe fest verbinden kann. Alternativ weist die Nadelschutzkappe keine Öffnung oder kein Befestigungsmittel auf, wobei sich das Befestigungselement des Eingriffselements in die Mantelfläche oder in die Kante der Nadelschutzkappe eingreifen oder einbohren kann.

Das Sperrelement kann dazu dienen, dass das Eingriffselement beim Abziehen der Kappe in die Eingriffsposition gelangt. Das Eingriffselement, insbesondere der lange Schenkel des hakenförmigen Eingriffselements kann entlang der ersten schrägen Fläche des Sperrelements gleiten und sich dabei plastisch verformen. Bei einer Axialbewegung oder einer kombinierten Axial-Drehbewegung der Kappe relativ zu dem Gehäuse der Injektionsvorrichtung bewegt sich die Kappe relativ zu der Nadelschutzkappe vorerst axial in die distale Richtung. Bei der Fortführung der Abziehbewegung der Kappe gelangt das Eingriffselement in Eingriff mit der Nadelschutzkappe, wobei das Sperrelement, insbesondere die erste schräge Fläche des Sperrelements bewirkt, dass das Eingriffselement in die unverformte Ausgestaltung gebracht wird, verformt wird oder radial nach innen verformt wird. Beispielsweise kann der lange Schenkel des hakenförmigen Eingriffselements dabei in die unverformte Ausgestaltung gebracht werden, verformt werden oder radial nach innen verformt werden, wobei der kurze Schenkel des hakenförmigen Eingriffselements radial nach innen ragt und in Eingriff mit der Nadelschutzkappe ist, insbesondere immer in Eingriff mit der Nadelschutzkappe ist. Nachdem das Eingriffselement in der Eingriffsposition ist und die Kappe weiter abgezogen wird, nimmt die Kappe die Nadelschutzkappe mit. Die Injektionsvorrichtung steht für eine Injektion zur Verfügung.

Alternativ kann das Sperrelement dazu dienen, beim Montieren der Injektionsvorrichtung das Eingriffselement der Kappe plastisch zu verformen. Beim Aufsetzen der Kappe auf das Gehäuse der Injektionsvorrichtung kann das Sperrelement, insbesondere die zweite schräge Fläche des Sperrelements des Gehäuses bewirken, dass das Eingriffselement der Kappe verformt, insbesondere radial nach innen verformt wird. Beispielsweise kann der lange Schenkel des hakenförmigen Eingriffselements verformt, insbesondere radial nach innen verformt werden, wobei der kurze Schenkel radial nach innen ragt. Dabei gleitet das Eingriffselement der Kappe über die schräge zweite Fläche des Sperrelements des Gehäuses. Am Ende der Aufsetzbewegung der Kappe kann sich das Eingriffselement verformen oder in die unverformte Ausgestaltung bringen bzw. gebracht werden oder kann radial nach aussen verformt werden. Das Eingriffselement der Kappe bewegt sich quer zur Längsachse (L), wobei sich das Eingriffselement proximal versetzt zu dem Sperrelement des Gehäuses positioniert. Wenn die Kappe auf dem Gehäuse der Injektionsvorrichtung aufgesetzt oder angebracht ist, ist das Eingriffselement der Kappe vorzugsweise unverformt ausgebildet. Beispielsweise ist der lange Schenkel des hakenförmigen Eingriffselements unverformt ausgebildet, wobei der kurze Schenkel radial nach innen ragt.

Bevorzugt kann ferner eine Nadelschutzhülse, welche dazu dient, vor oder nach erfolgter Injektion distal über das distale Ende der Injektionsnadel zu stehen, zumindest teilweise in das Gehäuse gesetzt werden, wobei an einem distalen Ende der Nadelschutzhülse die Kappe aufgesetzt wird. Die Kappe kann z. B. mit der Nadelschutzhülse reib- und/oder formschlüssig verbunden sein, wie z. B. verschnappt werden.

Ergänzend wird auf die Merkmale, die im Zusammenhang mit der hierin beschriebenen Vorrichtung offenbart werden, verwiesen, die auch die Vorrichtung für das Verfahren vorteilhaft weiterbilden.

Die Erfindung wurde anhand mehrerer Ausführungsformen und Beispiele beschrieben. Im Folgenden werden besonders bevorzugte Ausführungen der Erfindung anhand von Figuren beschrieben. Die dabei offenbarten Merkmale bilden die Erfindung einzeln und in jeglicher Merkmalskombination vorteilhaft weiter. Es zeigen:
- Figur 1: eine Längsschnittansicht einer ersten Ausführungsform einer erfindungsgemässen Injektionsvorrichtung, wobei eine Kappe (2) lösbar an der Injektionsvorrichtung angeordnet ist.
- Figuren 2a bis 2g: Längsschnittansichten des distalen Teils der Injektionsvorrichtungen aus Figur 1, wobei die einzelnen Montageschritte zur Montage der Kappe (2) an die Injektionsvorrichtung gemäss Figur 1 ersichtlich sind.
- Figuren 2h und 2i: Längsschnittansichten des distalen Teils der ersten Ausführungsform der Injektionsvorrichtung, wobei das Abziehen der Kappe (2) von der Injektionsvorrichtung ersichtlich ist.
- Figuren 3a bis 3d: Längsschnittansichten eines distalen Teils einer zweiten Ausführungsform einer erfindungsgemässen Injektionsvorrichtung, wobei die einzelnen Montageschritte zur Montage einer Kappe (2') an dieser Injektionsvorrichtung ersichtlich sind.
- Figuren 3e und 3f: Längsschnittansichten des distalen Teils der zweiten Ausführungsform der Injektionsvorrichtung, wobei das Abziehen der Kappe (2') von der Injektionsvorrichtung ersichtlich ist.
- Figuren 4a bis 4f: Längsschnittansichten eines distalen Teils einer dritten Ausführungsform einer erfindungsgemässen Injektionsvorrichtung, wobei die einzelnen Montageschritte zur Montage einer Kappe (2") an dieser Injektionsvorrichtung ersichtlich sind.
- Figuren 4g und 4h: Längsschnittansichten des distalen Teils der dritten Ausführungsform der Injektionsvorrichtung, wobei das Abziehen der Kappe (2") von der Injektionsvorrichtung ersichtlich ist.
- Figuren 5a bis 5c: Längsschnittansichten eines distalen Teils einer vierten Ausführungsform einer erfindungsgemässen Injektionsvorrichtung, wobei die einzelnen Montageschritte zur Montage einer Kappe (2''') an dieser Injektionsvorrichtung ersichtlich sind.
- Figuren 5d und 5e: Längsschnittansichten des distalen Teils der vierten Ausführungsform der Injektionsvorrichtung, wobei das Abziehen der Kappe (2''') von der Injektionsvorrichtung ersichtlich ist.
- Figur 6a: Perspektivenansicht einer ersten Ausführungsform eines Eingriffselements (2c"") mit einer Längsachse (L), wobei ein Entfernerelement (2d"") und das Eingriffselement (2c"") ersichtlich sind und das Eingriffselement (2c"") unverformt ist.
- Figur 6b: Perspektivenansicht entlang der Längsachse (L) der ersten Ausführungsform des Eingriffselements (2c"") gemäss Figur 6a.
- Figur 6c: Perspektivenansicht der ersten Ausführungsform des Eingriffselements (2c"") gemäss Figur 6b, wobei die Ansicht gegenüber Figur 6b um 90° gedreht ist.
- Figur 7a: Perspektivenansicht der ersten Ausführungsform des Eingriffselements (2c"") mit der Längsachse (L), wobei ein Entfernerelement (2d"") und das Eingriffselement (2c'''') ersichtlich sind und das Eingriffselement (2c"") plastisch radial nach aussen verformt ist.
- Figur 7b: Perspektivenansicht entlang der Längsachse (L) der ersten Ausführungsform des Eingriffselements (2c"") gemäss Figur 7a.
- Figur 7c: Perspektivenansicht der ersten Ausführungsform des Eingriffselements (2c"") gemäss Figur 7b, wobei die Ansicht gegenüber Figur 7b um 90° gedreht ist.
- Figur 8a: Perspektivenansicht einer zweiten Ausführungsform eines Eingriffselements (2c''''') mit einer Längsachse (L), wobei ein Entfernerelement (2d''''') und das Eingriffselement (2c''''') ersichtlich sind und das Eingriffselement (2c''''') unverformt ist.
- Figur 8b: Perspektivenansicht entlang der Längsachse (L) der zweiten Ausführungsform des Eingriffselements (2c''''') gemäss Figur 8a.
- Figur 8c: Perspektivenansicht der zweiten Ausführungsform des Eingriffselements (2c''''') gemäss Figur 8b, wobei die Ansicht gegenüber Figur 8b um 90° gedreht ist.
- Figur 9a: Perspektivenansicht der zweiten Ausführungsform des Eingriffselements (2c''''') mit der Längsachse (L), wobei ein Entfernerelement (2d''''') und das Eingriffselement (2c''''') ersichtlich sind und das Eingriffselement (2c''''') plastisch radial nach aussen verformt ist.
- Figur 9b: Perspektivenansicht entlang der Längsachse (L) der zweiten Ausführungsform des Eingriffselements (2c''''') gemäss Figur 9a.
- Figur 9c: Perspektivenansicht der zweiten Ausführungsform des Eingriffselements (2c''''') gemäss Figur 9b, wobei die Ansicht gegenüber Figur 9b um 90° gedreht ist.

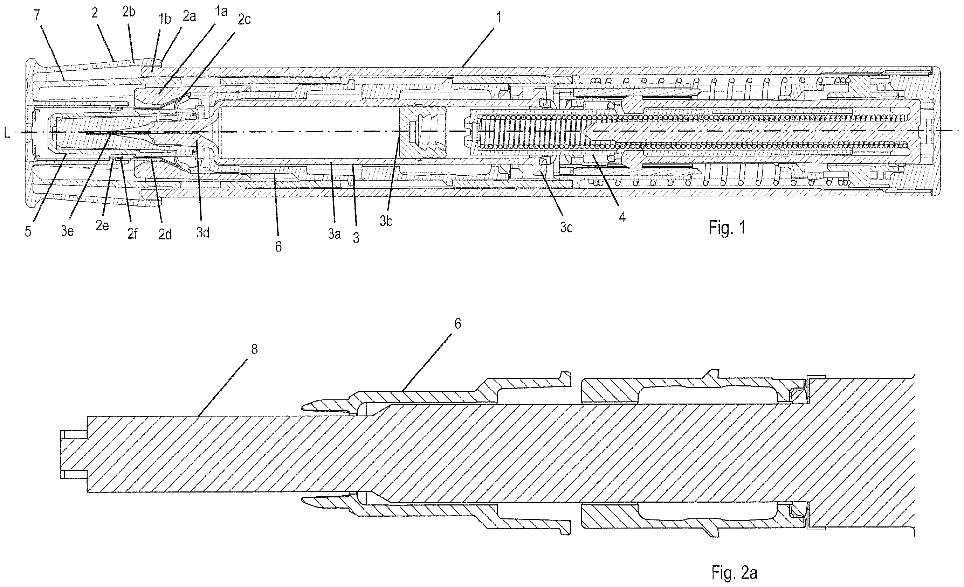

In der Figur 1 ist eine Längsschnittansicht einer ersten Ausführungsform einer erfindungsgemässen Injektionsvorrichtung ersichtlich, wobei eine Kappe (2) lösbar an der Injektionsvorrichtung angeordnet ist. Die Injektionsvorrichtung kann beispielsweise in einem Auslieferungszustand die Kappe (2) an dem distalen Ende aufgesetzt haben. Die Injektionsvorrichtung umfasst ein Gehäuse (1). Das Gehäuse (1) kann als hülsenförmiges, insbesondere zylindrisches Aufnahmegehäuse (1) mit einem distalen und einem proximalen Teil ausgebildet sein. An dem distalen Ende des Gehäuses (1) ist die Kappe (2) lösbar vorgesehen. Die Kappe (2) ist an dem distalen Ende des Gehäuses (1) oder alternativ an dem distalen Ende der Nadelschutzhülse (7) über eine formschlüssige Verbindung, insbesondere über eine Schnappverbindung lösbar befestigt. Die Kappe (2) weist ferner eine Hülse (2b) auf. Die Hülse (2b) kann vorzugsweise eine Innenhülse und eine Aussenhülse umfassen. Die Hülse (2b) kann vorzugsweise aus Kunststoff gebildet sein. Um die formschlüssige Verbindung zwischen der Kappe (2) und dem Gehäuse (1) zu bilden, kann die Kappe (2) ein oder mehrere Eingriffsglieder (2a) aufweisen, welche in an dem Gehäuse (1) der Injektionsvorrichtung entsprechend angeordnete Gegeneingriffsglieder (1b) eingreifen können, insbesondere verschnappt sein können. Die Hülse (2b) erstreckt sich wie das Gehäuse (1) entlang der Längsachse (L) der Injektionsvorrichtung. Durch eine Bewegung der Kappe (2) um und/oder entlang der Längsachse (L) kann die formschlüssige Verbindung, insbesondere die Schnappverbindung zwischen der Kappe (2) und dem Gehäuse (1) oder alternativ der Nadelschutzhülse (7) der Injektionsvorrichtung gelöst werden, wobei die Kappe (2) von dem Gehäuse (1) der Injektionsvorrichtung in die distale Richtung entfernt werden kann. Das distale Ende der Hülse (2b) ist im Wesentlichen verschlossen, sodass ein Zugriff von aussen in das Innere der Kappe (2) nicht oder nur erschwert möglich ist.

Die Kappe (2) umfasst ein Eingriffselement (2c). Das Eingriffselement (2c) ist plastisch verformbar. Das Eingriffselement (2c) ist vorzugsweise aus Metall, insbesondere aus Stahl, besonders bevorzugt aus rostfreiem Stahl oder aus rostfreiem Federstahl gebildet. Das Eingriffselement (2c) ist aus einem Material gebildet, welches eine Biegefestigkeit aufweist, welche eine plastische Verformung zulässt. Das Eingriffselement (2c) ist derart verformbar, dass es sich verformt, insbesondere radial nach aussen oder radial nach innen verformt oder eine unverformte Ausgestaltung einnimmt. Das Eingriffselement (2c) ist derart verformbar, dass es verformt, insbesondere radial nach aussen oder radial nach innen verformt oder unverformt wird oder ist. Das Eingriffselement (2c) ist hakenförmig ausgebildet, wobei das Eingriffselement (2c) einen langen und einen kurzen Schenkel aufweist.

Die Kappe (2) kann ferner ein Entfernerelement (2d) aufweisen. Das Entfernerelement (2d) ist vorzugsweise hülsenförmig ausgebildet, wobei an dem Entfernerelement (2d) das Eingriffselement (2c) vorgesehen sein kann. Das Entfernerelement (2d) und das Eingriffselement (2c) sind vorzugsweise einteilig und vorzugsweise aus dem gleichen Material gebildet. Das Entfernerelement (2d) und das Eingriffselement (2c) sind vorzugsweise aus einem Stanzbiegeteil gebildet. Das Stanzbiegeteil ist plastisch verformbar. Das Stanzbiegeteil ist vorzugsweise aus Metall, insbesondere aus Stahl, besonders bevorzugt aus rostfreiem Stahl, insbesondere aus rostfreiem Federstahl gebildet. Das Stanzbiegeteil ist aus einem Material gebildet, welches eine Biegefestigkeit aufweist, welche eine plastische Verformung zulässt.

Die Hülse (2b) kann das Eingriffselement (2c), insbesondere das Entfernerelement (2d) mit dem Eingriffselement (2c) zumindest teilweise umgeben. Die Hülse (2b) und das Entfernerelement (2d) mit dem Eingriffselement (2c) sind axialfest miteinander verbunden. Die Hülse (2b) und das Entfernerelement (2d) mit dem Eingriffselement (2c) sind zweiteilig ausgebildet. Das Entfernerelement (2d) ist mit der Hülse (2b) axialfest verbunden, insbesondere eingeschnappt, verklebt, umspritzt oder eingerastet. Dazu kann die Hülse (2b), insbesondere die Innenhülse der Hülse (2b) einen Vorsprung (2e) aufweisen, welcher in eine an dem Entfernerelement (2d) vorgesehene Ausnehmung (2f) eingerastet ist.

In dem Gehäuse (1) der Injektionsvorrichtung ist ein als Spritze ausgestalteter Produktbehälter (3) angeordnet. Der Produktbehälter (3) weist einen Produktbehälterabschnitt (3a) auf, der insbesondere hohlzylindrisch gebildet ist und dessen Innenwand mit einem in dem Produktbehälterabschnitt (3a) verschiebbar aufgenommenen Kolben (3b) einen Dichtspalt bildet, um so eine sterile Barriere zu bilden. An dem proximalen Ende des Produktbehälterabschnitts (3a) kann optional ein Flansch (3c), der auch als Fingerflansch bezeichnet wird, angeordnet sein. Der Produktbehälterabschnitt (3a) verjüngt sich an seinem distalen Ende zu einem Nadelhalteabschnitt (3d) hin, der einen deutlich geringeren Aussendurchmesser als der Produktbehälterabschnitt (3a) aufweist. Der Nadelhalteabschnitt (3d) umgibt den proximalen Teil einer Injektionsnadel (3e) und ist damit vorzugsweise unlösbar verbunden. Der Produktbehälter (3) weist eine fest verbundene Injektionsnadel (3e) auf. Die Injektionsnadel (3e) ragt von dem Nadelhalteabschnitt (3d) in die distale Richtung ab. Durch Verschieben des Kolbens (3b) in die distale Richtung kann das zwischen der Injektionsnadel (3d) und dem Kolben (3b) im Produktbehälterabschnitt (3a) angeordnete Produkt, vorzugweise flüssige Produkt durch die Injektionsnadel (3e) hindurch ausgegeben werden.

Um den Kolben (3b) des Produktbehälters (3) in die distale Richtung zu bewegen, kann in dem Gehäuse (1) der Injektionsvorrichtung eine Antriebseinheit vorgesehen sein. Alternativ kann die Antriebseinheit mit dem Gehäuse (1) der Injektionsvorrichtung kraft- und/oder form- und/oder stoffschlüssig verbunden sein. Die Antriebseinheit kann eine Kolbenstange (4) umfassen. Durch Verschieben der Kolbenstange (4) in die distale Richtung kann ein Produkt aus dem Produktbehälter (3) ausgeschüttet werden.

An dem Nadelhalteabschnitt (3d) ist eine Nadelschutzkappe (5) lösbar, wie z.B. form- und/oder reibschlüssig befestigt. Die Nadelschutzkappe (5) kann ein rigid needle shield (RNS) oder alternativ ein soft needle shield (SNS) sein. Die Nadelschutzkappe (5) umschliesst die Injektionsnadel (3e) und dichtet die Injektionsnadel (3e) gegenüber der Umgebung steril ab. Die Nadelschutzkappe (5) umgibt die Injektionsnadel (3e) derart, dass deren Sterilität in Bezug auf die Umgebung gewährleistet ist.

Das Eingriffselement (2c) der Kappe (2) ist derart verformbar, dass das Eingriffselement (2c) von einer beabstandeten Position, in welcher das Eingriffselement (2c) von der Nadelschutzkappe (5) radial beabstandet ist, in eine Eingriffsposition des Eingriffselements (2c), in welcher das Eingriffselement (2c) in Eingriff mit der Nadelschutzkappe (5) ist, gelangbar ist, wobei das Eingriffselement (2c) beim Entfernen der Kappe verformt wird.

Dadurch wird erreicht, dass beim Einfügen des Produktbehälters (3) in das Gehäuse (1) der Injektionsvorrichtung keine oder sehr wenige Kräfte, insbesondere keine oder sehr wenige von dem Eingriffselement (2c) ausgeübte Kräfte auf die Nadelschutzkappe (5) wirken.

An dem Gehäuse (1) der Injektionsvorrichtung oder an einem Gehäuse (1) fest verbundenen Teil der Injektionsvorrichtung ist ein Sperrelement (1a) vorgesehen. Das Sperrelement (1a) weist mindestens eine erste schräge Fläche auf. Die erste schräge Fläche des Sperrelements (1a) ragt nach innen. Die erste schräge Fläche des Sperrelements (1a) weist eine Neigung auf. Alternativ kann das Sperrelement (1a) an dem Produktbehälterhalter (3) vorgesehen sein. Beim Abziehen der Kappe (2) von dem Gehäuse (1) kann das Eingriffselement (2c) der Kappe (2) mit dem Sperrelement (1a), insbesondere mit der ersten schrägen Fläche des Sperrelements (1a) des Gehäuses (1) derart zusammenwirken, dass das Eingriffselement (2c) der Kappe (2) in die Eingriffsposition gelangt, wobei das Eingriffselement (2c) der Kappe (2) in Eingriff mit der Nadelschutzkappe (5) gelangt. Das Sperrelement (1a) des Gehäuses (1) hält oder bringt das Eingriffselement (2c) der Kappe (2) in die Eingriffsposition des Eingriffselements (2c).

Der Produktbehälter (3) kann optional in einem z.B. hülsenförmigen Produktbehälterhalter (6) angeordnet sein. Der verjüngende Abschnitt des Produktbehälterabschnitts (3a) kann sich beispielsweise an einer nach innen ragenden Schulter des Produktbehälterhalters (6) in die distale Richtung abstützen. Alternativ kann sich der Flansch (3c) des Produktbehälters (3) an dem Produktbehälterhalter (6) in die distale Richtung abstützen. Noch weiter alternativ kann der Produktbehälterhalter (6) den Produktbehälter (3) an seinem Produktbehälterabschnitt (3a) reibschlüssig halten. Der Produktbehälterhalter (6) kann z.B. axialfest oder verschiebbar in dem Gehäuse (1) der Injektionsvorrichtung angeordnet sein.

In dem Gehäuse (1) der Injektionsvorrichtung kann optional eine Nadelschutzhülse (7) angeordnet sein, die in Bezug auf das Gehäuse (1) der Injektionsvorrichtung zum Auslösen einer Produktausschüttung in die proximale Richtung verschiebbar ist und nach erfolgter Produktausschüttung in die distale Richtung verschiebbar ist, um die Spitze der Injektionsnadel (3e) abzudecken, um eine Verletzungsgefahr zur verringern. Ferner oder alternativ kann die Hülse (2) z. B. über eine Schnappverbindung mit der Nadelschutzhülse (7) lösbar verbunden sein. Solche Nadelschutzhülsen (7) sind aus dem Stand der Technik bekannt und können als vorteilhafte Weiterbildung der Erfindung angesehen werden.

Die erfindungsgemässe Injektionsvorrichtung kann irgendeine Ausgestaltung aufweisen, vorausgesetzt, dass die Injektionsvorrichtung in einem Gehäuse (1) einen Produktbehälter (3) umfasst. Die Injektionsvorrichtung kann als einen aus dem Stand der Technik bekannten Autoinjektor, bei welchem ein automatisches Ausschütten des Produkts erfolgt, ausgestaltet sein. Es können aber auch andere Injektionsvorrichtungen vorgesehen sein.

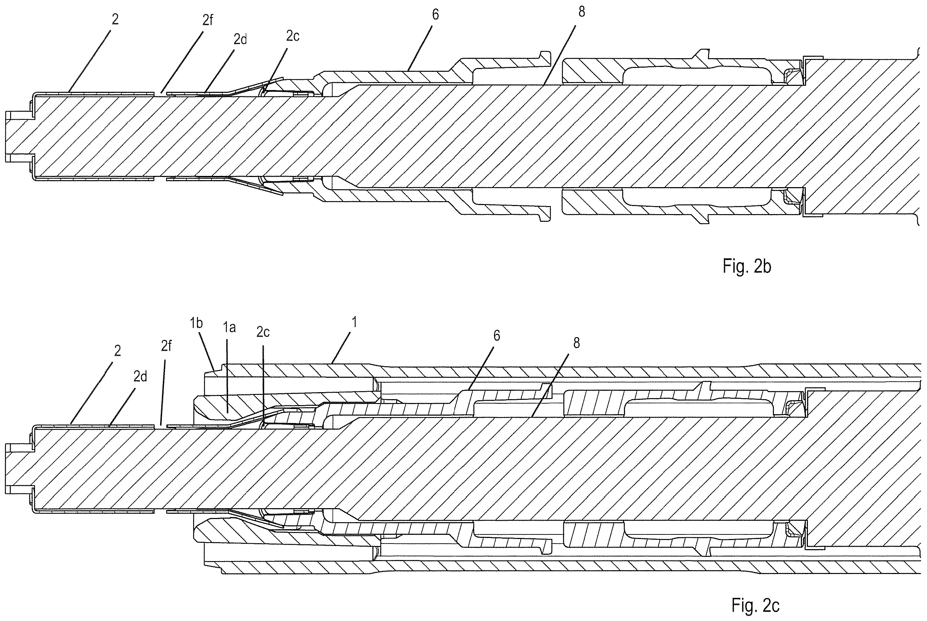

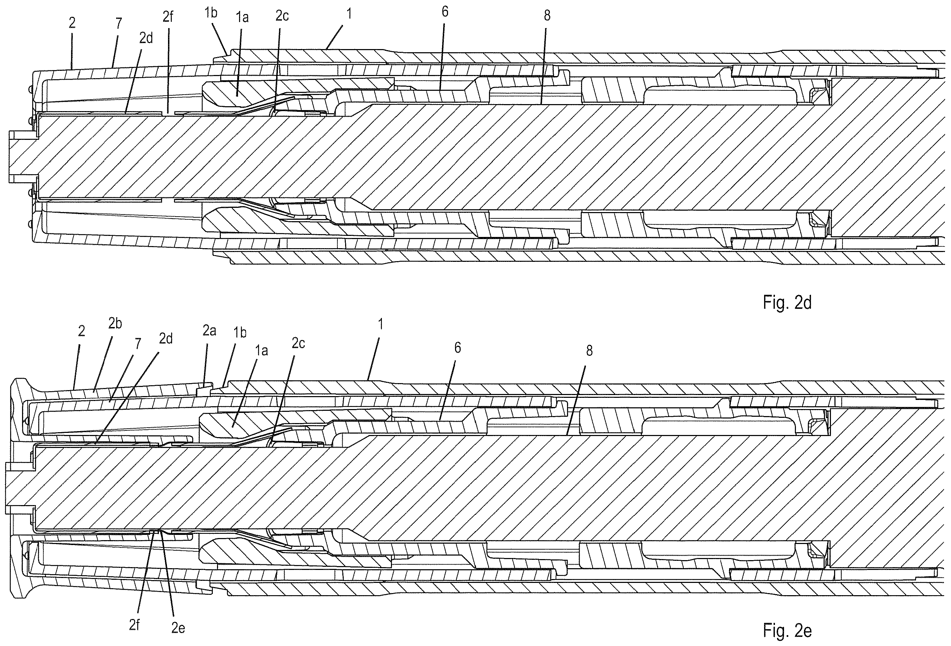

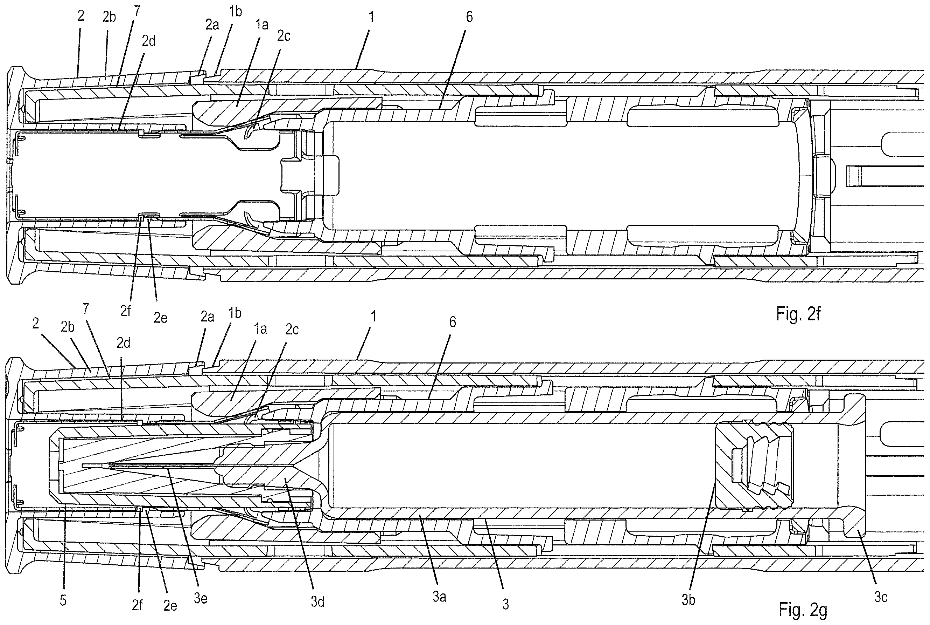

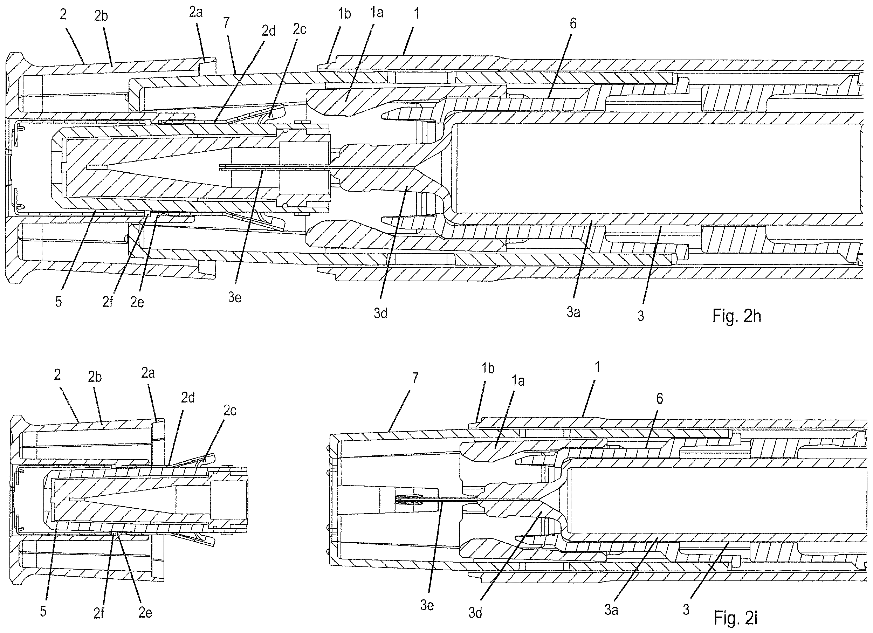

In den Figuren 2a bis 2g sind Längsschnittansichten des distalen Teils der Injektionsvorrichtungen aus Figur 1 ersichtlich, wobei die einzelnen Montageschritte zur Montage der Kappe (2) an die Injektionsvorrichtung gemäss Figur 1 dargestellt sind. Ferner ist in den Figuren 2h und 2i Längsschnittansichten des distalen Teils der Injektionsvorrichtung aus Figur 1 ersichtlich, wobei das Abziehen der Kappe (2) von der Injektionsvorrichtung dargestellt ist. Der hülsenförmige Produktbehälterhalter (6) wird auf ein Montagewerkzeug (8), insbesondere auf einen Montagewerkzeugdorn (8) gesetzt. Der Produktbehälterhalter (6) wird dabei von dem distalen Ende des Montagewerkzeugs (8) relativ zu dem Montagewerkzeug (8) verschoben. Der Produktbehälterhalter (6) wird auf dem Montagewerkzeug (8) in die proximale Richtung verschoben, bis der Produktbehälterhalter (6) eine proximale Position einnimmt. Der Produktbehälterhalter (6) dient zur Aufnahme des Produktbehälters (3). Das hülsenförmige Entfernerelement (2d) der Kappe (2), welches ein Eingriffselement (2c) umfasst, wird danach auf das Montagewerkzeug (8) gesetzt. Das Eingriffselement (2c) der Kappe (2) ist plastisch radial nach aussen verformt. Der lange Schenkel des hakenförmigen Eingriffselements (2c) ist plastisch radial nach aussen verformt, wobei der kurze Schenkel des hakenförmigen Eingriffselements (2c) radial nach innen ragt. Das radial nach aussen verformte Eingriffselement (2c) gleitet beim Verschieben relativ zu dem Montagewerkzeug (8) in die proximale Richtung über das distale Ende des Produktbehälterhalters (6), wobei das Entfernerelement (2d) eine distale Position einnimmt. Der plastisch radial nach aussen verformte lange Schenkel und/oder der kurze Schenkel des Eingriffselements (2c) ist auf dem distalen Ende des Produktbehälters (6) abgestützt. Diese Abstützung dient dazu, dass kein vorzeitiger Eingriff zwischen dem Eingriffselement (2c) und der Nadelschutzkappe (5) erfolgt. Danach wird das Gehäuse (1) auf das Montagewerkzeug (8) gesetzt. Das Gehäuse (1) wird relativ zu dem Montagewerkzeug (8) in die proximale Richtung verschoben, bis das an dem Gehäuse (1) vorgesehene Sperrelement (1a), insbesondere die erste schräge Fläche des Sperrelements (1a) in den Bereich des radial nach aussen verformten Eingriffselements (2c), insbesondere in den Bereich des radial nach aussen verformten langen Schenkels des hakenförmigen Eingriffselements (2c) kommt, aber vorzugsweise nicht berührt. Das radial nach aussen verformte Eingriffselement (2c) und die erste schräge Fläche des Sperrelements (1a) sind etwa parallel zueinander angeordnet. Alternativ können der Produktbehälterhalter (6) und das Gehäuse (1) einteilig ausgebildet sein, wobei der Schritt des Aufsetzens des Produktbehälterhalters (6) weggelassen werden kann. Ferner kann eine Nadelschutzhülse (7) auf das Montagewerkzeug (8) aufgesetzt werden, wobei die Nadelschutzhülse (7) von dem distalen Ende des Montagewerkzeugs (8) relativ zu dem Montagewerkzeug (8) verschoben wird. Die Nadelschutzhülse (7) wird zumindest teilweise in das Gehäuse (1) der Injektionsvorrichtung gesetzt. Alternativ kann die Injektionsvorrichtung keine Nadelschutzhülse (7) umfassen, wobei dieser Schritt weggelassen werden kann. Danach kann die Hülse (2b) der Kappe (2) auf das Montagewerkzeug (8) gesetzt werden. Die Hülse (2b) der Kappe (2) wird an dem distalen Ende des Gehäuses (1) angebracht. Die Hülse (2b) wird insbesondere über eine Schnappverbindung lösbar mit dem Gehäuse (1) oder alternativ mit der Nadelschutzhülse (7) verbunden. Die Hülse (2b), insbesondere die Aussenhülse der Hülse (2b) umfasst ein oder mehrere Eingriffsglieder (2a), welche in an dem Gehäuse (1) entsprechend vorgesehene Gegeneingriffsglieder (1b) eingreifen können. Der an der Hülse (2b), insbesondere der an der Innenhülse der Hülse (2b) vorgesehene Vorsprung (2e) rastet in die an dem Entfernerelement (2d) vorgesehene Ausnehmung (2f) ein. Die Hülse (2b) ist mit dem Entfernerelement (2d) axialfest verbunden. Die Hülse (2b), insbesondere die Aussenhülse der Hülse (2b) umgibt teilweise die Nadelschutzhülse (7). Die Hülse (2b) ist an dem distalen Ende der Nadelschutzhülse (7) aufgesetzt. Danach wird das Montagewerkzeug (8) entfernt. Die Baugruppe bestehend aus mindestens dem Gehäuse (1) und der Kappe (2), wobei die Kappe (2) das Eingriffselement (2c) umfasst, kann optional zur weiteren Montage an einen anderen Montageort geliefert werden. Um die Injektionsvorrichtung herzustellen, kann der Produktbehälter (3) in den Produktbehälterhalter (6) oder in das Gehäuse (1) der Injektionsvorrichtung gesetzt werden. Dabei werden keine oder sehr wenige Kräfte von dem Eingriffselement (2c) der Kappe (2) auf die Nadelschutzkappe (5), welche auf dem Produktbehälter (2) lösbar angeordnet ist, ausgeübt. Die Injektionsnadel (3e) des Produktbehälters (3) wird durch die Nadelschutzkappe (5) gegenüber der Umgebung steril abgedichtet. Der mit einem Produkt vorgefüllte Produktbehälter (3) wird von einem proximalen Ende des Gehäuses (1) in das Gehäuse (1) eingesetzt. Der Produktbehälter (3) wird bis zum Erreichen einer distalen Endposition relativ zu dem Produktbehälterhalter (6) oder zu dem Gehäuse (1) in die distale Richtung verschoben. Das Eingriffselement (2c) der Kappe (2) ist in der beabstandeten Position, in welcher das Eingriffselement (2c) von der Nadelschutzkappe (5), welche an dem Produktbehälter (3) angeordnet ist, radial beabstandet ist. Danach kann eine Antriebseinheit in das Gehäuse (1) der Injektionsvorrichtung gesetzt werden. Die Antriebseinheit kann von dem proximalen Ende des Gehäuses (1) in das Gehäuse (1) eingesetzt werden. Alternativ kann die Antriebseinheit mit dem Gehäuse (1) der Injektionsvorrichtung kraft- und/oder form- und/oder stoffschlüssig verbunden sein. Die Injektionsvorrichtung ist montiert. Die Injektionsvorrichtung ist in dem Auslieferungszustand. Um die Injektionsvorrichtung zu benutzen, wird die Kappe (2) von dem distalen Ende des Gehäuses (1) der Injektionsvorrichtung entfernt. Dabei wird die formschlüssige Verbindung zwischen der Hülse (2b), insbesondere der Aussenhülse der Hülse (2b) der Kappe (2), insbesondere dem Eingriffsglied (2a) der Hülse (2b) und dem Gehäuse (1), insbesondere dem Gegeneingriffsglied (1b) des Gehäuses (1) gelöst. Aufgrund der axialfesten Verbindung zwischen der Hülse (2b) und dem Entfernerelement (2d), wobei das Entfernerelement (2d) mit dem Eingriffselement (2c) axialfest verbunden ist, bewegt sich die Hülse (2b) relativ zu dem Gehäuse (1) in die distale Richtung. Bei dieser Bewegung gleitet das Eingriffselement (2c), insbesondere der lange Schenkel des hakenförmigen Eingriffselements (2c) der Kappe (2) über die erste schräge Fläche des Sperrelements (1a) des Gehäuses (1) der Injektionsvorrichtung. Das Eingriffselement (2c) wird dabei derart verformt, dass das Eingriffselement (2c) in Eingriff mit der Nadelschutzkappe (5) gelangt. Das Eingriffselement (2c) der Kappe (2) gelangt in die Eingriffsposition, in welcher das Eingriffselement (2c) in Eingriff mit der Nadelschutzkappe (5) ist, wobei das Eingriffselement (2c) beim Entfernen der Kappe (2) plastisch verformt wird. Das Eingriffselement (2c), insbesondere der lange Schenkel des hakenförmigen Eingriffselements (2c) gelangt dabei bevorzugt in eine unverformte oder in eine radial nach innen verformte Ausgestaltung, wobei das Eingriffselement (2c), insbesondere der kurze Schenkel des hakenförmigen Eingriffselements (2c) eine axialfeste Verbindung mit der Nadelschutzkappe (5) eingeht. Das Eingriffselement (2c), insbesondere der kurze Schenkel des hakenförmigen Eingriffselements (2c) greift oder bohrt sich dabei in eine Mantelfläche der Nadelschutzkappe (5) ein. Bei Fortführung der Abziehbewegung wird die Nadelschutzkappe (5) von der Kappe (2) mitgenommen, wobei die Nadelschutzkappe (5) von dem Produktbehälter (3) entfernt wird und in der Kappe (2) gehalten wird. Die Nadelschutzkappe (5) ist von der Kappe (2) aufgenommen. Die Injektionsvorrichtung kann zur Verabreichung des Produkts benutzt werden.

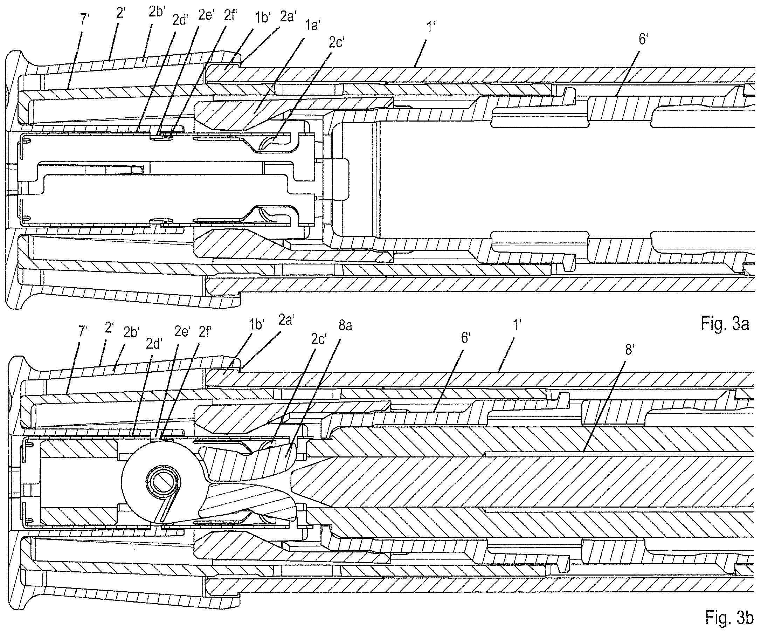

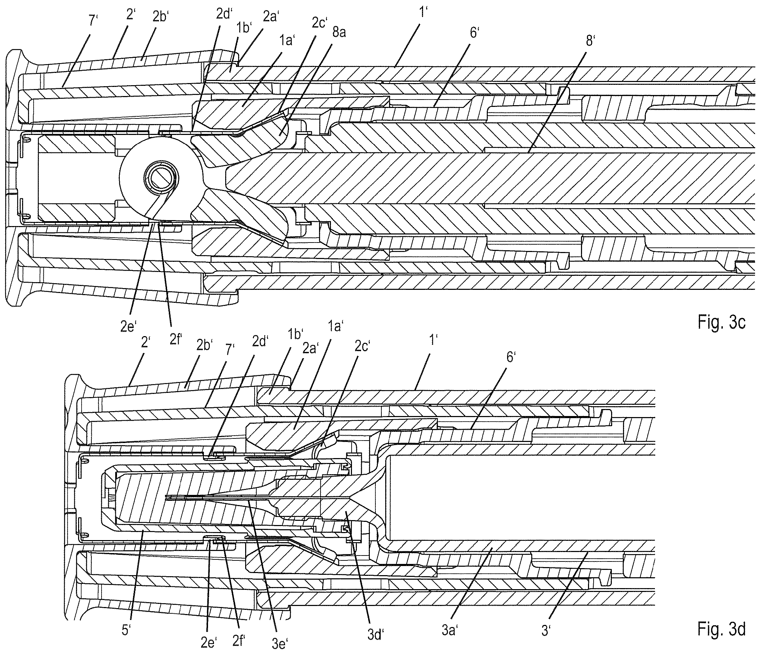

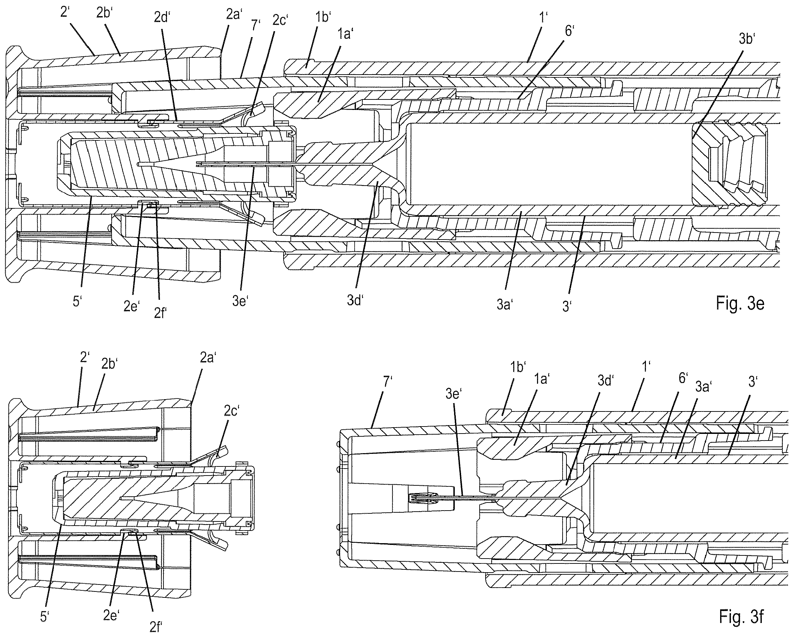

In den Figuren 3a bis 3d sind Längsschnittansichten eines distalen Teils einer zweiten Ausführungsform einer erfindungsgemässen Injektionsvorrichtung ersichtlich, wobei die einzelnen Montageschritte zur Montage einer Kappe (2') an dieser Injektionsvorrichtung dargestellt sind. Ferner sind in den Figuren 3e und 3f Längsschnittansichten des distalen Teils der zweiten Ausführungsform der Injektionsvorrichtung ersichtlich, wobei das Abziehen der Kappe (2') von der Injektionsvorrichtung dargestellt ist. In mehreren Montageschritten werden in ein Gehäuse (1') der Injektionsvorrichtung ein Produktbehälterhalter (6') und eine Nadelschutzhülse (7') angeordnet. Alternativ kann das Gehäuse (1') und der Produktbehälter (6') einteilig ausgebildet sein, wobei der Schritt des Einsetzens des Produktbehälterhalters (6') in das Gehäuse (1') weggelassen werden kann. Alternativ kann die Injektionsvorrichtung keine Nadelschutzhülse (7') aufweisen, wobei der Schritt des Einsetzens der Nadelschutzhülse (7') in das Gehäuse (1') weggelassen werden kann. An dem Gehäuse (1') oder an einem Gehäuse (1') fest verbundenen Teil ist ein Sperrelement (1a') vorgesehen. Das Sperrelement (1a') weist mindestens eine erste schräge Fläche auf. Die erste schräge Fläche ragt nach innen. Die erste schräge Fläche weist eine Neigung auf. An einem distalen Ende des Gehäuses (1) wird ferner eine Kappe (2') angeordnet. Die Kappe (2') umfasst eine Hülse (2b'), ein Entfernerelement (2d') und ein Eingriffselement (2c'). Die Hülse (2b') kann eine Aussenhülse und eine Innenhülse umfassen. Die Hülse (2b'), das Entfernerelement (2d') und das Eingriffselement (2c') sind vorzugweise axialfest miteinander verbunden. Die Hülse (2b') umgibt teilweise das Eingriffselement (2c'), insbesondere das Entfernerelement (2d') und das Eingriffselement (2c'). Das Entfernerelement (2d') und das Eingriffselement (2c') sind vorzugweise einteilig ausgebildet, wobei die Hülse (2b') ein separates Teil bildet. Das Entfernerelement (2d') und das Eingriffselement (2c') sind vorzugsweise aus einem Stanzbiegeteil gebildet. Das Entfernerelement (2d') ist vorzugsweise über eine Vorsprung-Ausnehmung-Verbindung (2e', 2f') mit der Hülse (2b') axialfest verbunden. Das Eingriffselement (2c') ist vorzugsweise aus Metall, insbesondere aus Stahl, besonders bevorzugt aus rostfreiem Stahl oder Federstahl gebildet. Das Eingriffselement (2c') oder das Stanzbiegeteil ist aus einem Material gebildet, welches eine Biegefestigkeit aufweist, welche eine plastische Verformung zulässt. Das Eingriffselement (2c') ist derart verformbar, dass es sich verformt, insbesondere radial nach aussen oder radial nach innen verformt oder eine unverformte Ausgestaltung einnimmt. Das Eingriffselement (2c') ist derart verformbar, dass es verformt, insbesondere radial nach aussen oder radial nach innen verformt oder unverformt wird oder ist. Das Eingriffselement (2c') ist hakenförmig ausgebildet. Das hakenförmige Eingriffselement (2c') umfasst einen langen und einen kurzen Schenkel. Der kurze Schenkel des hakenförmigen Eingriffselements (2c') ragt gegen das Innere der Injektionsvorrichtung in die Richtung der Längsachse (L) der Injektionsvorrichtung. Wenn die Kappe (2') auf das distale Ende des Gehäuses (1') gesetzt wird, ist das Eingriffselement (2c'), insbesondere der lange Schenkel des hakenförmigen Eingriffselements (2c') unverformt ausgestaltet. Beim Aufsetzen der Kappe (2') auf das Injektionsgerät bewegt sich das Eingriffselement (2c') axial relativ zu dem Sperrelement (1a') des Gehäuses (1') in die proximale Richtung, wobei das Eingriffselement (2c') nicht plastisch verformt wird. Ein an der Hülse (2b') der Kappe (2') vorgesehenes Eingriffsglied (2a') greift in ein an dem Gehäuse angeordnetes Gegeneingriffsglied (1b') ein. Um die Injektionsvorrichtung weiter zu montieren, wird von dem proximalen Ende des Gehäuses (1') der Injektionsvorrichtung ein Montagewerkzeug (8'), insbesondere ein Spreizdorn (8') in die Injektionsvorrichtung geführt. Der Spreizdorn (8') umfasst ein oder mehrere Biegeelemente (8a), welche das Eingriffselement (2c') verformen können, insbesondere radial nach aussen verformen können. Das Eingriffselement (2c') der Kappe (2') wird parallel zu der ersten schrägen Fläche des Sperrelements (1a') des Gehäuses (1) plastisch verformt. Dabei wird das Biegeelement (8a) des Spreizdorns (8') von der Längsachse (L) der Injektionsvorrichtung quer zur Längsachse (L) der Injektionsvorrichtung und danach wieder in die Längsachse (L) bewegt. Das Montagewerkzeug (8'), insbesondere der Spreizdorn (8') wird aus der Injektionsvorrichtung gezogen. Die Baugruppe bestehend aus mindestens dem Gehäuse (1') und der Kappe (2'), wobei die Kappe (2') das Eingriffselement (2c') umfasst, kann optional zur weiteren Montage an einen anderen Montageort geliefert werden. Ein Produktbehälter (3') kann in den Produktbehälterhalter (6') oder in das Gehäuse (1') der Injektionsvorrichtung gesetzt werden. Aufgrund des radial nach aussen verformten Eingriffselements (2c') der Kappe (2') werden beim Einsetzen des Produktbehälters (3') mit der Nadelschutzkappe (5') keine oder sehr wenige Kräfte von dem Eingriffselement (2c') der Kappe (2') auf die Nadelschutzkappe (5') ausgeübt. Eine an dem Produktbehälter (3') angeordnete Injektionsnadel (3e') wird durch die Nadelschutzkappe (5') gegenüber der Umgebung steril abgedichtet. Der mit einem Produkt vorgefüllte Produktbehälter (3') wird von einem proximalen Ende des Gehäuses (1') in das Gehäuse (1') eingesetzt. Der Produktbehälter (3') wird bis zum Erreichen einer distalen Endposition relativ zu dem Produktbehälterhalter (6') oder zu dem Gehäuse (1') in die distale Richtung verschoben. Das Eingriffselement (2c') der Kappe (2') ist in der beabstandeten Position, in welcher das Eingriffselement (2c') von der Nadelschutzkappe (5'), welche an dem Produktbehälter (3') angeordnet ist, radial beabstandet ist. Danach kann eine Antriebseinheit in das Gehäuse (1') der Injektionsvorrichtung gesetzt werden. Die Antriebseinheit kann von dem proximalen Ende des Gehäuses (1') in das Gehäuse (1') eingesetzt werden. Alternativ kann die Antriebseinheit mit dem Gehäuse (1') der Injektionsvorrichtung kraft- und/oder form- und/oder stoffschlüssig verbunden sein. Die Injektionsvorrichtung ist montiert. Die Injektionsvorrichtung ist in dem Auslieferungszustand. Zur Benutzung der Injektionsvorrichtung wird die Kappe (2') von dem distalen Ende des Gehäuses (1') der Injektionsvorrichtung entfernt. Die formschlüssige Verbindung zwischen der Hülse (2b') der Kappe (2'), insbesondere dem Eingriffsglied (2a') der Hülse (2b') und dem Gehäuse (1), insbesondere dem Gegeneingriffsglied (1b') des Gehäuses wird dabei gelöst. Die Kappe (2') bewegt sich relativ zu dem Gehäuse (1) in die distale Richtung. Bei dieser Bewegung gleitet das Eingriffselement (2c'), insbesondere der lange Schenkel des hakenförmigen Eingriffselements (2c') der Kappe (2') über die erste schräge Fläche des Sperrelements (1a') des Gehäuses (1') der Injektionsvorrichtung. Das Eingriffselement (2a') wird derart verformt, dass das Eingriffselement (2c') in Eingriff mit der Nadelschutzkappe (5') gelangt. Das Eingriffselement (2c') der Kappe (2') gelangt in die Eingriffsposition, in welcher das Eingriffselement (2c') in Eingriff mit der Nadelschutzkappe (5') ist, wobei das Eingriffselement (2c') beim Entfernen der Kappe plastisch verformt wird. Das Eingriffselement (2c') gelangt dabei bevorzugt in eine unverformte oder in eine radial nach innen verformte Ausgestaltung. Das Eingriffselement (2c') ist in axialfester Verbindung mit der Nadelschutzkappe (5'). Das Eingriffselement (2c'), insbesondere der kurze Schenkel des hakenförmigen Eingriffselements (2c') greift oder bohrt sich in eine Mantelfläche der Nadelschutzkappe (5') ein. Die Nadelschutzkappe (5') wird von der Kappe (2') mitgenommen, wobei die Nadelschutzkappe (5') von dem Produktbehälter (3') entfernt wird und in der Kappe (2') gehalten wird. Die Injektionsvorrichtung kann zur Verabreichung des Produkts benutzt werden.

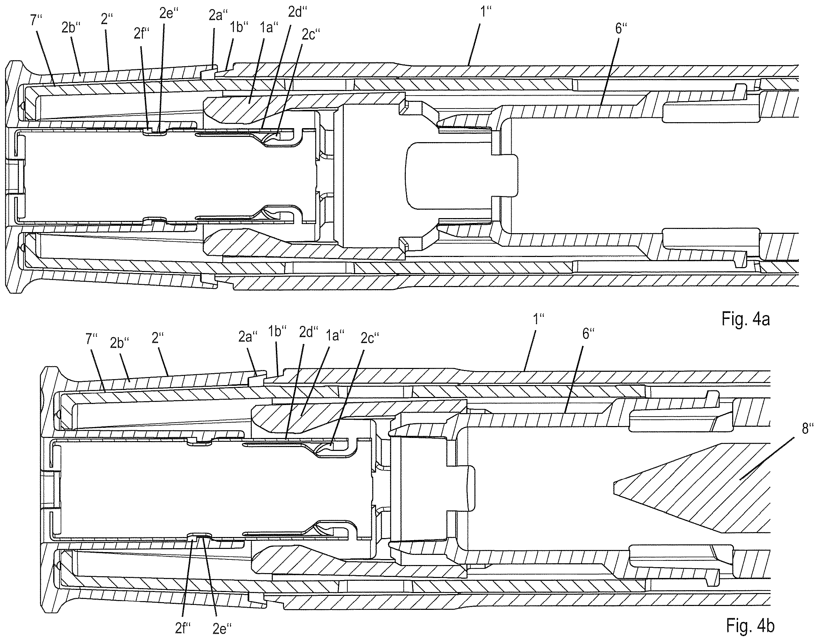

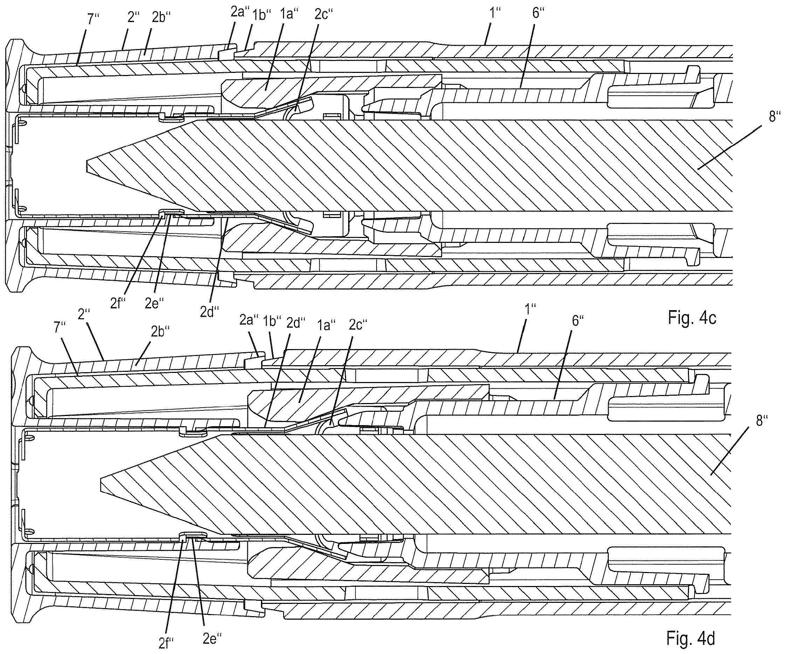

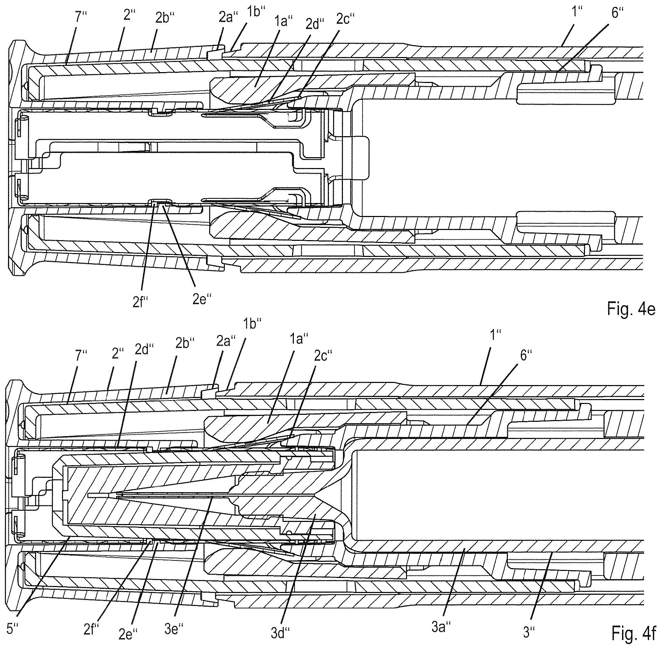

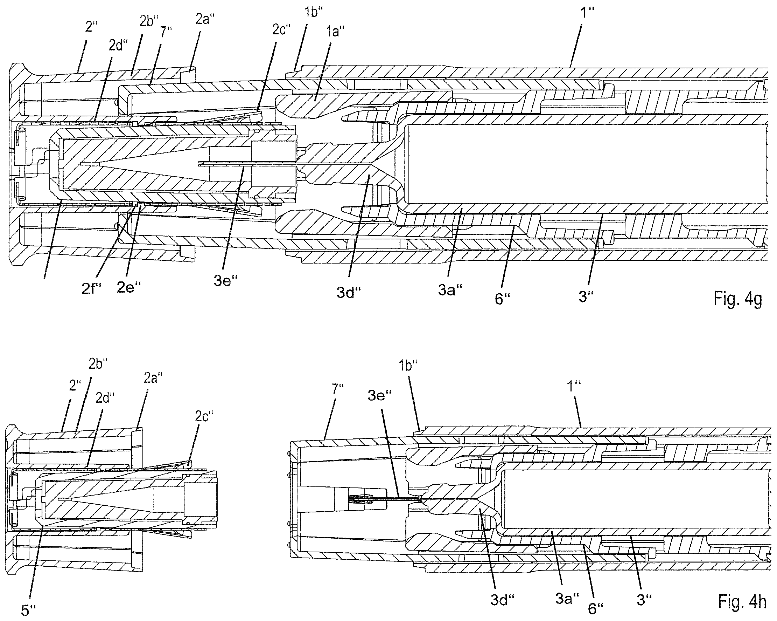

In den Figuren 4a bis 4f sind Längsschnittansichten eines distalen Teils einer dritten Ausführungsform einer erfindungsgemässen Injektionsvorrichtung ersichtlich, wobei die einzelnen Montageschritte zur Montage der Kappe (2") an dieser Injektionsvorrichtung dargestellt sind. Ferner sind in den Figuren 4g und 4h Längsschnittansichten des distalen Teils der dritten Ausführungsform der Injektionsvorrichtung ersichtlich, wobei das Abziehen der Kappe (2") von der Injektionsvorrichtung dargestellt ist. Vorerst werden in ein Gehäuse (1") der Injektionsvorrichtung ein Produktbehälterhalter (6") und eine Nadelschutzhülse (7") eingesetzt. In einer anderen Ausführungsform der Erfindung kann alternativ das Gehäuse (1") und der Produktbehälter (6") einteilig ausgebildet sein, wobei der Schritt des Einsetzens des Produktbehälterhalters (6") in das Gehäuse (1") weggelassen werden kann. In einer weiteren Ausführungsform kann die Injektionsvorrichtung keine Nadelschutzhülse (7") aufweisen, wobei der Schritt des Einsetzens der Nadelschutzhülse (7") in das Gehäuse (1') weggelassen werden kann. An dem Gehäuse (1") oder an einem Gehäuse (1") fest verbundenen Teil ist ein Sperrelement (1a") angeordnet, wobei das Sperrelement (1a") mindesten eine erste schräge Fläche umfasst. Die erste schräge Fläche des Sperrelements (1a") ragt nach innen. Die erste schräge Fläche weist eine Neigung auf. In einem weiteren Schritt wird eine Kappe (2") an einem distalen Ende des Gehäuses (1") angebracht. Die Kappe (2") weist vorzugsweise eine Hülse (2b"), ein Entfernerelement (2d") und ein Eingriffselement (2c") auf. Die Hülse (2b"), das Entfernerelement (2d") und das Eingriffselement (2c") sind vorzugweise axialfest miteinander verbunden. Alternativ können die Hülse (2b"), das Entfernerelement (2d") und das Eingriffselement (2c") einteilig ausgebildet sein. Die Hülse (2b") umgibt teilweise das Eingriffselement (2c"), insbesondere das Entfernerelement (2d") und das Eingriffselement (2c"). Das Entfernerelement (2d") und das Eingriffselement (2c") sind vorzugsweise einteilig ausgebildet, wobei die Hülse (2b") ein separates Teil bildet. Das Entfernerelement (2d") und das Eingriffselement (2c") sind vorzugsweise aus einem Stanzbiegeteil gebildet. Das Entfernerelement (2d") ist vorzugsweise über eine Vorsprung-Ausnehmung-Verbindung (2e", 2f") mit der Hülse (2b") axialfest verbunden. Das Eingriffselement (2c") ist vorzugsweise aus Metall, insbesondere aus Stahl, besonders bevorzugt aus rostfreiem Stahl oder Federstahl gebildet. Das Eingriffselement (2c") ist aus einem Material gebildet, welches eine Biegefestigkeit aufweist, welche eine plastische Verformung zulässt. Das Eingriffselement (2c") ist derart verformbar, dass es sich verformt, insbesondere radial nach aussen oder radial nach innen verformt oder eine unverformte Ausgestaltung einnimmt. Das Eingriffselement (2c") ist derart verformbar, dass es verformt, insbesondere radial nach aussen oder radial nach innen verformt oder unverformt wird oder ist. Das Eingriffselement (2c") kann hakenförmig ausgebildet sein. Das hakenförmige Eingriffselement (2c") weist einen langen und einen kurzen Schenkel auf. Bei dem Anbringen der Kappe (2") auf das distale Ende des Gehäuses (1") ist das Eingriffselement (2c"), insbesondere der lange Schenkel des hakenförmigen Eingriffselements (2c") unverformt ausgestaltet. Beim Aufsetzen der Kappe (2") auf das distale Ende der Injektionsvorrichtung bewegt sich das Eingriffselement (2c") relativ zu dem Sperrelement (1a") des Gehäuses (1") in die proximale Richtung, wobei das Eingriffselement (2c") nicht plastisch verformt wird. Am Ende der Aufsetzbewegung der Kappe (2") greift ein an der Hülse (2b") vorgesehenes Eingriffsglied (2a") in ein an dem Gehäuse angeordnetes Gegeneingriffsglied (1b") ein. In einem weiteren Schritt wird von dem proximalen Ende des Gehäuses (1") der Injektionsvorrichtung ein Montagewerkzeug (8"), insbesondere ein Spreizdorn (8") in die Injektionsvorrichtung eingeführt. Ein oder mehrere Biegeelemente des Spreizdorns (8") verformt das Eingriffselement (2c"). Das Eingriffselement (2c") wird durch den Spreizdorn (8") derart radial nach aussen verformt, dass das Eingriffselement (2c") etwa parallel zu der ersten schrägen Fläche des Sperrelements (1a") des Gehäuses (1') plastisch verformt ist. Dabei wird das Biegeelement des Spreizdorns (8") von der Längsachse (L) der Injektionsvorrichtung quer zur Längsachse (L) der Injektionsvorrichtung und danach wieder in die Längsachse (L) bewegt. Nach der plastischen Verformung des Eingriffselements (2c") der Kappe (2") wird der Spreizdorn (8") wird aus der Injektionsvorrichtung geführt. Besonders bevorzugt kann vor oder nach dem Entfernen des Montagewerkzeugs (8"), insbesondere dem Spreizdorn (8") der Produktbehälterhalter (6") derart relativ zu dem Gehäuse (1") in die distale Richtung verschoben werden, dass das plastisch verformte Eingriffselement (2c"), insbesondere der lange Schenkel des hakenförmigen Eingriffselements(2c") der Kappe (2") über ein distales Ende des Produktbehälterhalters (6") zu liegen kommt. Der plastisch radial nach aussen verformte lange Schenkel und/oder der kurze Schenkel des Eingriffselements (2c") ist auf dem distalen Ende des Produktbehälters (6") abgestützt. Diese Abstützung dient dazu, dass kein vorzeitiger Eingriff zwischen dem Eingriffselement (2c") und der Nadelschutzkappe (5") erfolgt. Das distale Ende des Produktbehälterhalters (6") kann das Eingriffselement (2c") in der verformten Ausgestaltung halten. Die Baugruppe, welche mindestens das Gehäuse (1") und die Kappe (2") umfasst, kann optional zur weiteren Montage an einen anderen Montageort geliefert werden. Ein mit einem Produkt vorgefüllte Produktbehälter (3") kann in den Produktbehälterhalter (6") oder in das Gehäuse (1") der Injektionsvorrichtung gesetzt werden. Aufgrund des radial nach aussen verformten Eingriffselements (2c") der Kappe (2") werden beim Einsetzen des Produktbehälters (3") mit der Nadelschutzkappe (5") keine oder sehr wenige Kräfte von dem Eingriffselement (2c") der Kappe (2") auf die Nadelschutzkappe (5") ausgeübt. Eine Injektionsnadel (3e") des Produktbehälters (3") wird durch die Nadelschutzkappe (5") gegenüber der Umgebung steril abgedichtet. Der Produktbehälter (3") wird von einem proximalen Ende des Gehäuses (1") in das Gehäuse (1") eingesetzt, wobei der Produktbehälter (3") bis zum Erreichen einer distalen Endposition relativ zu dem Produktbehälterhalter (6") oder zu dem Gehäuse (1") in die distale Richtung verschoben wird. Dabei ist das Eingriffselement (2c") der Kappe (2") in der beabstandeten Position, in welcher das Eingriffselement (2c") von der Nadelschutzkappe (5") radial beabstandet ist. In einem weiteren Schritt kann eine Antriebseinheit in das Gehäuse (1") der Injektionsvorrichtung eingefügt werden. Die Antriebseinheit kann ebenfalls von dem proximalen Ende des Gehäuses (1") in das Gehäuse (1") eingesetzt werden. In anderen Ausführungsformen kann die Antriebseinheit mit dem Gehäuse (1") der Injektionsvorrichtung kraft- und/oder form- und/oder stoffschlüssig verbunden werden. Die Injektionsvorrichtung befindet sich in dem Auslieferungszustand. Zur Benutzung der Injektionsvorrichtung wird die Kappe (2") von dem distalen Ende des Gehäuses (1") der Injektionsvorrichtung entfernt. Dabei wird die formschlüssige Verbindung zwischen der Hülse (2b") der Kappe (2") und dem Gehäuse (1") gelöst. Die Kappe (2") bewegt sich relativ zu dem Gehäuse (1") in die distale Richtung. Bei dieser Axialbewegung oder kombinierten Axial-Dreh-Bewegung gleitet das Eingriffselement (2c"), insbesondere der lange Schenkel des hakenförmigen Eingriffselements (2c") der Kappe (2") über die erste schräge Fläche des Sperrelements (1a") des Gehäuses (1") der Injektionsvorrichtung. Das Eingriffselement (2c") wird derart verformt, dass das Eingriffselement (2c") in Eingriff mit der Nadelschutzkappe (5") gelangt. Alternativ verformt sich das Eingriffselement (2c") ohne Einwirkung des Sperrelements (1a"), insbesondere in die unverformte Ausgestaltung. Das Eingriffselement (2c") der Kappe (2") gelangt in die Eingriffsposition, in welcher das Eingriffselement (2c") in Eingriff mit der Nadelschutzkappe (5") ist, wobei das Eingriffselement (2c") beim Entfernen der Kappe (2") verformt wird. Das Eingriffselement (2c") gelangt dabei bevorzugt in eine unverformte oder in eine radial nach innen verformte Ausgestaltung. Das Eingriffselement (2c") ist in axialfester Verbindung mit der Nadelschutzkappe (5"). Das Eingriffselement (2c"), insbesondere der kurze Schenkel des hakenförmigen Eingriffselements (2c") greift oder bohrt sich in eine Mantelfläche der Nadelschutzkappe (5") ein. Die Nadelschutzkappe (5") wird von der Kappe (2") mitgenommen. Die Injektionsvorrichtung kann zur Verabreichung des Produkts benutzt werden.

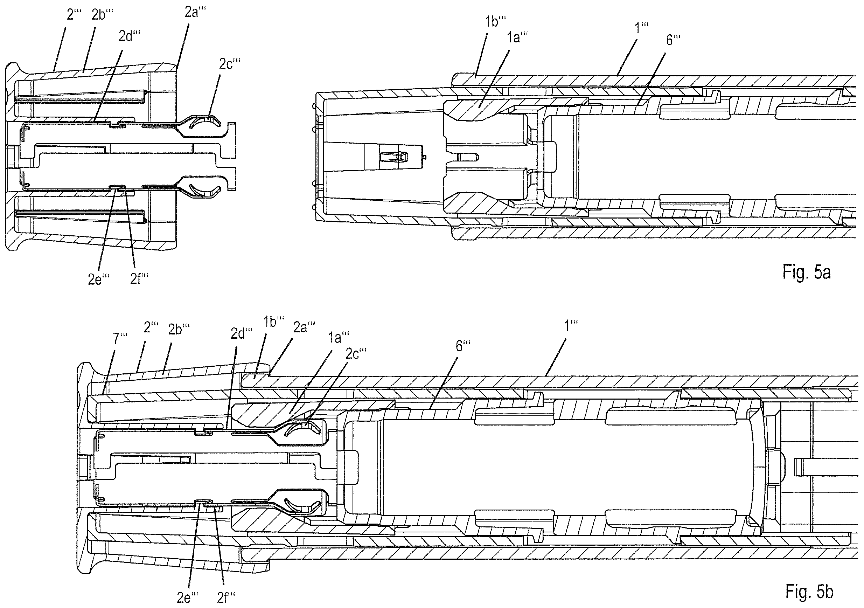

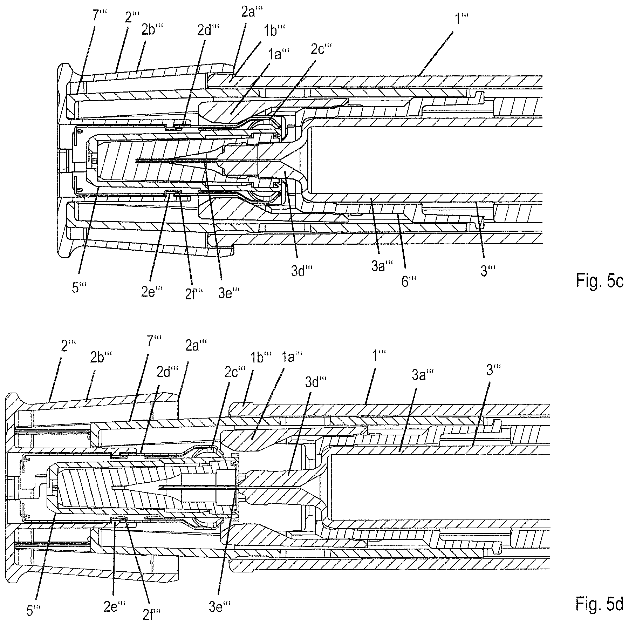

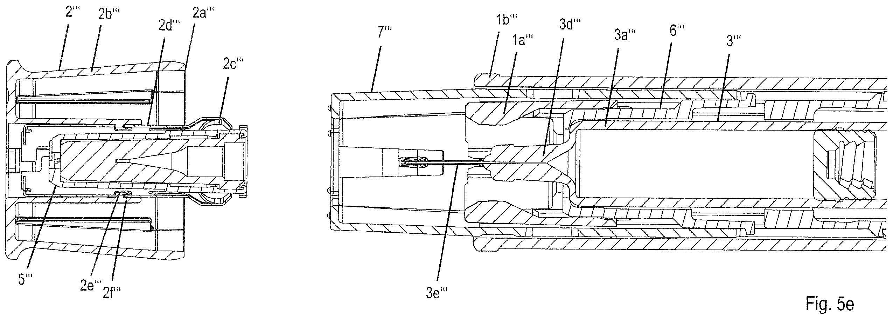

In den Figuren 5a bis 5c sind Längsschnittansichten eines distalen Teils einer vierten Ausführungsform einer erfindungsgemässen Injektionsvorrichtung ersichtlich, wobei die einzelnen Montageschritte zur Montage der Kappe an dieser Injektionsvorrichtung dargestellt sind. Ferner sind in den Figuren 5d und 5e Längsschnittansichten des distalen Teils der vierten Ausführungsform der Injektionsvorrichtung ersichtlich, wobei das Abziehen der Kappe (2''') von der Injektionsvorrichtung dargestellt ist. In einem Schritt wird ein Gehäuse (1'''), welches ein Produktbehälterhalter (6''') und eine Nadelschutzhülse (6''') aufweist, bereitgestellt. Alternativ kann das Gehäuse (1''') und der Produktbehälterhalter (6''') einteilig ausgebildet sein. In einem weiteren Ausführungsbeispiel kann die Injektionsvorrichtung keine Nadelschutzhülse (7''') aufweisen, wobei zumindest ein Gehäuse (1''') der Injektionsvorrichtung bereitgestellt wird. An dem Gehäuse (1''') oder an einem Gehäuse (1''') fest verbundenen Teil ist ein Sperrelement (1a''') vorgesehen. Das Sperrelement (1a''') weist mindestens eine erste und eine zweite schräge Fläche auf. Die erste und die zweite schräge Flächen ragen nach innen. Die erste und die zweite schräge Flächen weisen je eine Neigung auf. Die erste und die zweite schräge Fläche sind zueinander geneigt. Ferner wird eine Kappe (2''') bereitgestellt. Die Kappe (2''') umfasst eine Hülse (2b'''), ein Entfernerelement (2d''') und ein Eingriffselement (2c'''). Die Hülse (2b'''), das Entfernerelement (2d''') und das Eingriffselement (2c''') sind vorzugweise axialfest miteinander verbunden. Die Hülse (2b''') umgibt teilweise das Eingriffselement (2c'''), insbesondere das Entfernerelement (2d''') mit dem Eingriffselement (2c'''). Das Entfernerelement (2d') und das Eingriffselement (2c''') sind vorzugweise einteilig ausgebildet, wobei die Hülse (2b''') ein separates Teil bildet. Das Entfernerelement (2d''') und das Eingriffselement (2c''') sind vorzugsweise aus einem Stanzbiegeteil gebildet. Das hülsenförmige Entfernerelement (2d''') ist vorzugsweise über eine Vorsprung-Ausnehmung-Verbindung (2e''', 2f''') mit der Hülse (2b''') axialfest verbunden. Das Eingriffselement (2c''') oder das Stanzbiegeteil ist vorzugsweise aus Metall, insbesondere aus Stahl, besonders bevorzugt aus rostfreiem Stahl oder Federstahl gebildet. Das Eingriffselement (2c''') oder das Stanzbiegeteil ist aus einem Material gebildet, welches eine Biegefestigkeit aufweist, welche eine plastische Verformung zulässt. Das Eingriffselement (2c''') ist derart verformbar, dass es sich verformt, insbesondere radial nach aussen oder radial nach innen verformt oder eine unverformte Ausgestaltung einnimmt. Das Eingriffselement (2c''') ist derart verformbar, dass es verformt, insbesondere radial nach aussen oder radial nach innen verformt oder unverformt wird oder ist. Das Eingriffselement (2c''') kann hakenförmig ausgebildet sein. Die Kappe (2''') wird auf ein distales Ende des Gehäuses (1''') gesetzt, wobei das Eingriffselement (2c'''), insbesondere der lange Schenkel des hakenförmigen Eingriffselements (2c''') unverformt ausgestaltet ist. Beim Aufsetzen der Kappe (2''') auf das distale Ende der Injektionsvorrichtung wird das Eingriffselement (2c''') relativ zu dem Sperrelement (1a''') des Gehäuses (1''') in die proximale Richtung bewegt, wobei das Eingriffselement (2c'''), insbesondere der lange Schenkel des hakenförmigen Eingriffselements (2c''') radial nach innen elastisch verformt wird. Wenn das Eingriffselement (2c''') in eine Position gelangt, welche proximal versetzt zu dem Sperrelement (1a''') des Gehäuses (1''') ist, nimmt das Eingriffselement (2c''') wieder seine unverformte Ausgestaltung an. Gleichzeitig oder bei Weiterführung der Bewegung greift ein an der Hülse (2b''') der Kappe (2''') vorgesehenes Eingriffsglied (2a''') in ein an dem Gehäuse angeordnetes Gegeneingriffsglied (1b''') ein und bildet eine lösbare Verbindung. Die Baugruppe bestehend aus mindestens dem Gehäuse (1''') und der Kappe (2'''), wobei die Kappe (2''') das Eingriffselement (2c''') umfasst, kann optional zur weiteren Montage an einen anderen Montageort geliefert werden. Ein mit einem Produkt vorgefüllte Produktbehälter (3''') kann in den Produktbehälterhalter (6''') oder in das Gehäuse (1''') der Injektionsvorrichtung gesetzt werden. Aufgrund des unverformten Eingriffselements (2c''') der Kappe (2''') werden beim Einsetzen des Produktbehälters (3''') mit der Nadelschutzkappe (5''') keine oder sehr wenige Kräfte von dem Eingriffselement (2c''') der Kappe (2''') auf die Nadelschutzkappe (5''') ausgeübt. Eine an dem Produktbehälter (3''') vorgesehene Injektionsnadel (3e''') wird durch die Nadelschutzkappe (5''') gegenüber der Umgebung steril abgedichtet. Der mit einem Produkt vorgefüllte Produktbehälter (3''') wird von einem proximalen Ende des Gehäuses (1''') in das Gehäuse (1''') eingesetzt. Der Produktbehälter (3''') wird bis zum Erreichen einer distalen Endposition relativ zu dem Produktbehälterhalter (6''') oder zu dem Gehäuse (1''') in die distale Richtung verschoben. Das Eingriffselement (2c''') der Kappe (2''') ist in der beabstandeten Position, in welcher das Eingriffselement (2c''') von der Nadelschutzkappe (5''') radial beabstandet ist. In das Gehäuse (1''') der Injektionsvorrichtung kann durch einen weiteren Schritt eine Antriebseinheit gesetzt werden. Die Antriebseinheit kann von dem proximalen Ende des Gehäuses (1''') in das Gehäuse (1''') eingesetzt werden. Alternativ kann die Antriebseinheit mit dem Gehäuse (1''') der Injektionsvorrichtung kraft- und/oder form- und/oder stoffschlüssig verbunden werden. Die Injektionsvorrichtung ist montiert. Die Injektionsvorrichtung ist in dem Auslieferungszustand. Um die Injektionsvorrichtung zu benutzen, wird die Kappe (2''') von dem distalen Ende des Gehäuses (1''') der Injektionsvorrichtung entfernt. Die formschlüssige Verbindung zwischen der Hülse (2b''') der Kappe (2''') und dem Gehäuse (1''') wird dabei gelöst. Die Kappe (2''') bewegt sich relativ zu dem Gehäuse (1''') in die distale Richtung. Bei dieser Bewegung gleitet das Eingriffselement (2c'''), insbesondere der lange Schenkel des hakenförmigen Eingriffselements (2c''') der Kappe (2''') über die erste schräge Fläche des Sperrelements (1a''') des Gehäuses (1''') der Injektionsvorrichtung. Das Eingriffselement (2a''') wird derart plastisch verformt, dass das Eingriffselement (2c''') in Eingriff mit der Nadelschutzkappe (5''') gelangt. Das Eingriffselement (2c''') der Kappe (2''') gelangt in die Eingriffsposition, in welcher das Eingriffselement (2c''') in Eingriff mit der Nadelschutzkappe (5''') ist, wobei das Eingriffselement (2c''') beim Entfernen der Kappe verformt wird. Das Eingriffselement (2c''') verformt sich plastisch radial nach innen. Das Eingriffselement (2c''') umfasst ferner vorzugsweise ein oder mehrere Befestigungselemente, wobei das Befestigungselement derart ausgebildet ist, dass es eine feste, insbesondere eine axial- und radialfeste Verbindung mit einer Nadelschutzkappe (5'''), insbesondere mit einer Mantelfläche der Nadelschutzkappe (5''') eingehen kann. Die Nadelschutzkappe (5''') wird folglich bei der Abziehbewegung der Kappe (2''') von der Kappe (2''') mitgenommen. Die Injektionsvorrichtung kann zur Verabreichung des Produkts benutzt werden.

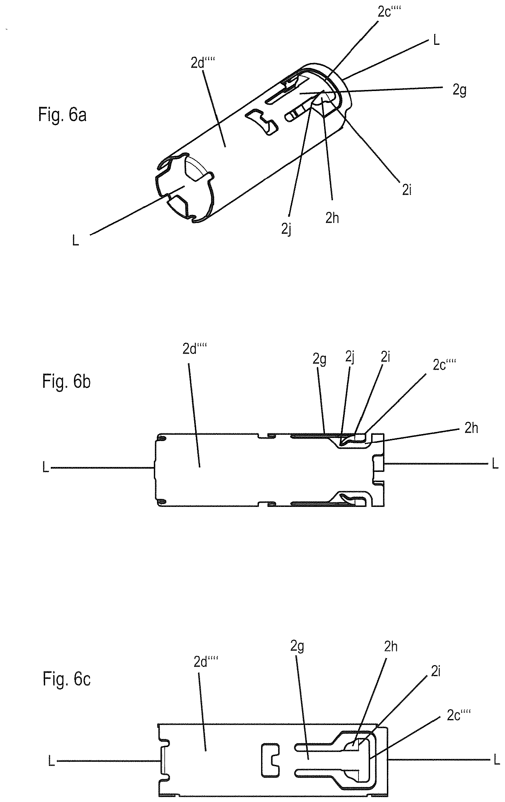

In der Figur 6a ist eine Perspektivenansicht einer ersten Ausführungsform eines Eingriffselements (2c"") mit einer Längsachse (L), wobei ein Entfernerelement (2d"") und das Eingriffselement (2c"") ersichtlich sind und das Eingriffselement (2c"") unverformt ist, dargestellt. Ferner ist in der Figur 6b eine Perspektivenansicht entlang der Längsachse (L) der ersten Ausführungsform des Eingriffselements (2c"") gemäss Figur 6a ersichtlich. Des Weiteren wird in der Figur 6c eine Perspektivenansicht der ersten Ausführungsform des Eingriffselements (2c"") gemäss Figur 6b, wobei die Ansicht gegenüber Figur 6b um 90° gedreht ist, gezeigt. Diese erste Ausführungsform des Eingriffselements (2c"") kann für alle bereits erwähnten Ausführungsformen einer erfindungsgemässen Injektionsvorrichtungen und/oder kann für alle bereits erwähnten Verfahren, insbesondere zum Montieren einer Injektionsvorrichtung und/oder Vorbereiten einer Injektionsvorrichtung für die Verabreichung eines Produkts verwendet werden. Das Eingriffselement (2c"") ist in diesem Ausführungsbeispiel mit dem Entfernerelement (2d"") axialfest verbunden, wobei das Entfernerelement (2d"") mit einer Hülse (nicht ersichtlich) einer Kappe (nicht ersichtlich) axialfest verbunden, insbesondere eingeschnappt, verklebt, umspritzt oder eingerastet sein kann. In alternativen Ausführungsformen kann das Eingriffselement (2c"") mit einer Kappe (nicht ersichtlich) oder mit einer Hülse (nicht ersichtlich) axialfest verbunden sein, wobei kein Entfernerelement (2d"") vorgesehen ist. In diesem Ausführungsbeispiel ist das Entfernerelement (2c"") hülsenförmig ausgebildet. Besonders bevorzugt sind das Entfernerelement (2c"") und das Eingriffselement (2c"") aus einem Stanzbiegeteil gebildet. Das Stanzbiegeteil ist plastisch verformbar. Das Stanzbiegeteil ist vorzugsweise aus Metall, insbesondere aus Stahl, besonders bevorzugt aus rostfreiem Stahl, insbesondere aus rostfreiem Federstahl gebildet. Das Eingriffselement (2d"") umfasst einen langen (2g) und einen kurzen Schenkel (2h). Der lange (2g) und der kurze Schenkel (2h) sind miteinander verbunden. Der lange Schenkel (2g) ist unverformt ausgebildet. Der lange Schenkel (2g) erstreckt sich entlang der Längsachse (L). Der kurze Schenkel (2h) ragt radial nach innen. Der kurze Schenkel (2h) ist zahn- oder dreieckförmig oder spitzwinklig ausgebildet. Der kurze Schenkel (2h) umfasst vorzugsweise gerade (2i) und gekrümmte Seiten. Vorzugsweise umfasst der kurze Schenkel (2h) zwei gekrümmte und eine gerade Seite (2i). Eine Spitze (2j) des dreieck- oder dreieckförmigen oder spitzwinkligen kurzen Schenkels (2h) ragt radial nach innen. Vorzugsweise ist die Spitze (2j) gegenüber der geraden Seite (2i) des kurzen Schenkels (2h) angeordnet. Die Spitze (2j) des zahn- oder dreieckförmigen oder spitzwinklig kurzen Schenkels (2h) ist derart ausgebildet, dass die Spitze (2j) in eine Nadelschutzkappe (nicht ersichtlich) eingreifen kann. Die Spitze (2j) ist vorzugsweise krallenförmig ausgebildet. Der lange (2g) und der kurze Schenkel (2h) des Eingriffselements (2c"") sind derart miteinander plastisch verformt verbunden, dass sich der kurze Schenkel (2h) von dem langen Schenkel (2g) unter einem Winkel, insbesondere unter einem Winkel von weniger als 90° quer zur Längsachse (L) radial nach innen erstreckt. Der lange (2g) und der kurze Schenkel (2h) des Eingriffselements (2c"") sind derart miteinander verbunden, dass die gerade Seite (2i) des zahn- oder dreieckförmigen oder spitzwinklig kurzen Schenkel (2h) unter einem Winkel, insbesondere unter einem Winkel von 90° quer zur Längsachse (L) mit dem langen Schenkel (2g) verbunden ist und die Spitze (2j) des kurzen Schenkels (2j) des Eingriffselement (2c"") radial nach innen ragt. Der lange Schenkel (2g) weist mehrere, insbesondere zwei kurze Schenkel (2h) auf, wobei die zwei kurzen Schenkel (2h) um die Längsachse (L) in Umfangsrichtung angeordnet sind. Ferner umfasst das Entfernerelement (2d"") mehrere, insbesondere zwei lange Schenkel (2g), wobei die zwei langen Schenkel (2g) einander gegenüber an dem Entfernerelement (4d"") oder alternativ an der Kappe (nicht ersichtlich) oder an der Hülse (nicht ersichtlich) vorgesehen sind.

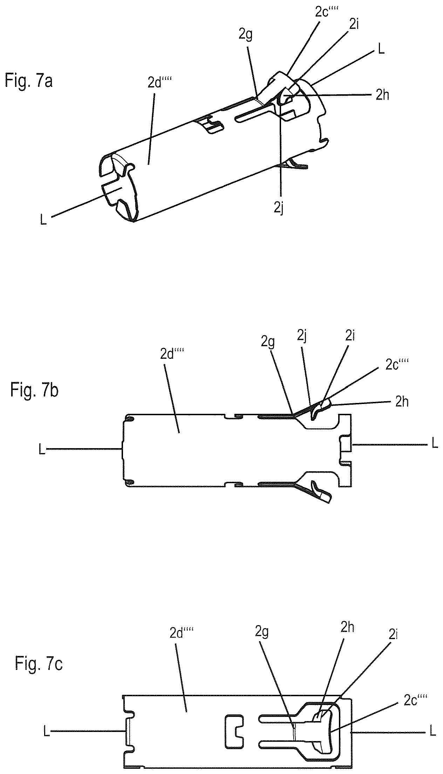

In der Figur 7a ist eine Perspektivenansicht der ersten Ausführungsform des Eingriffselements (2c"") mit der Längsachse (L), wobei ein Entfernerelement (2d"") und das Eingriffselement (2c"") ersichtlich sind und das Eingriffselement (2c"") plastisch radial nach aussen verformt ist, dargestellt. Ferner ist in der Figur 7b eine Perspektivenansicht entlang der Längsachse (L) der ersten Ausführungsform des Eingriffselements (2c"") gemäss Figur 7a ersichtlich. Des Weiteren wird in der Figur 7c eine Perspektivenansicht der ersten Ausführungsform des Eingriffselements (2c"") gemäss Figur 7b, wobei die Ansicht gegenüber Figur 7b um 90° gedreht ist, gezeigt. Der lange Schenkel (2g) des Eingriffselements (2c"") ist unter einem Winkel quer zur Längsachse (L) verformbar. In diesem Ausführungsbeispiel ist der lange Schenkel (2g) des Eingriffselements (2c'''') unter einem Winkel quer zur Längsachse (L) unter einem Winkel von weniger als 90° quer zur Längsachse (L) plastisch radial nach aussen verformt. In einer alternativen Ausführungsform kann der lange Schenkel (2g) des Eingriffselements (2c"") unter einem Winkel quer zur Längsachse (L) unter einem Winkel von weniger als 90° quer zur Längsachse (L) plastisch radial nach innen verformt sein. Der kurze Schenkel (2h) ragt radial nach innen.

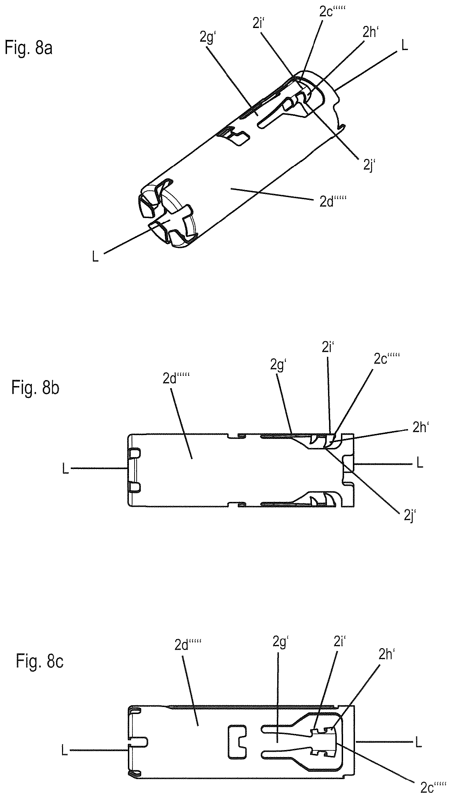

In der Figur 8a ist eine Perspektivenansicht einer zweiten Ausführungsform eines Eingriffselements (2c''''') mit einer Längsachse (L), wobei ein Entfernerelement (2d''''') und das Eingriffselement (2c''''') ersichtlich sind und das Eingriffselement (2c''''') unverformt ist, dargestellt. Ferner ist in der Figur 8b eine Perspektivenansicht entlang der Längsachse (L) der zweiten Ausführungsform des Eingriffselements (2c''''') gemäss Figur 8a ersichtlich. Des Weiteren wird in der Figur 8c eine Perspektivenansicht der zweiten Ausführungsform des Eingriffselements (2c''''') gemäss Figur 8b, wobei die Ansicht gegenüber Figur 8b um 90° gedreht ist, gezeigt. Diese zweite Ausführungsform des Eingriffselements (2c''''') kann für alle bereits erwähnten Ausführungsformen einer erfindungsgemässen Injektionsvorrichtungen und/oder kann für alle bereits erwähnten Verfahren, insbesondere zum Montieren einer Injektionsvorrichtung und/oder Vorbereiten einer Injektionsvorrichtung für die Verabreichung eines Produkts verwendet werden. Das Eingriffselement (2c''''') unterscheidet sich von dem ersten Ausführungsbeispiel des Eingriffselements (2c"") durch die Anordnung und/oder die Ausgestaltung des langen (2g') und des kurzen Schenkels (2h') des Eingriffselements (2c'''''). Der lange Schenkel (2g') erstreckt sich entlang der Längsachse (L). Der lange Schenkel (2g') ist unverformt ausgebildet. Der kurze Schenkel (2h') ist zahn- oder dreieckförmig oder spitzwinklig ausgebildet. Der zahn- oder dreieckförmige oder spitzwinklige kurze Schenkel (2h') weist gerade (2i') und gekrümmte Seiten auf. Vorzugsweise umfasst der kurze Schenkel (2h') zwei gekrümmte und eine gerade Seite (2i'). Eine Spitze (2j') des zahn- oder dreieckförmigen oder spitzwinkligen kurzen Schenkels (2h') ragt radial nach innen. Vorzugsweise ist die Spitze (2j') gegenüber der geraden Seite (2i') des kurzen Schenkels (2h') angeordnet. Die Spitze (2j') des zahn- oder dreieckförmigen oder spitzwinkligen kurzen Schenkels (2h') ist derart ausgebildet, dass die Spitze (2j') in eine Nadelschutzkappe (nicht ersichtlich) eingreifen kann. Die Spitze (2j') ist vorzugsweise krallenförmig ausgebildet. Die Spitze (2j') des kurzen Schenkels (2h') ragt nach innen. Der lange (2g') und der kurze Schenkel (2h') des Eingriffselements (2c''''') sind derart miteinander plastisch verformt verbunden, dass sich der kurze Schenkel (2h') von dem langen Schenkel (2g') unter einem Winkel, insbesondere unter einem Winkel zur Längsachse (L) entlang der Längsachse (L) radial nach innen erstreckt. Der lange (2g') und der kurze Schenkel (2h') des Eingriffselements (2c''''') sind derart miteinander verbunden, dass die gerade Seite (2i') des zahn- oder dreieckförmigen oder spitzwinkligen kurzen Schenkels entlang der Längsachse (L) mit dem langen Schenkel (2h') verbunden ist und die Spitze (2j') des kurzen Schenkels des Eingriffselements radial nach innen ragt. An dem langen Schenkel (2g') sind mehrere kurze Schenkel vorgesehen. Die mehreren, insbesondere zwei der kurzen Schenkel (2g') sind hintereinander und entlang der Längsachse (L) angeordnet. Zusätzlich sind mehrere, insbesondere zwei der kurzen Schenkel (2g') um die Längsachse (L) in Umfangsrichtung angeordnet. Die mehreren kurzen Schenkel können verschieden ausgestaltet sein. Die Abstände zwischen der geraden Seite (2i") und der Spitze (2j") des zahn- oder dreieckförmigen oder spitzwinkligen kurzen Schenkels (2h') sind verschieden, insbesondere die Abstände der kurzen Schenkel, welche hintereinander angeordnet sind. Ferner umfasst das Entfernerelement (2d''''') mehrere, insbesondere zwei lange Schenkel (2g'), wobei die zwei langen Schenkel (2g') einander gegenüber angeordnet sind.

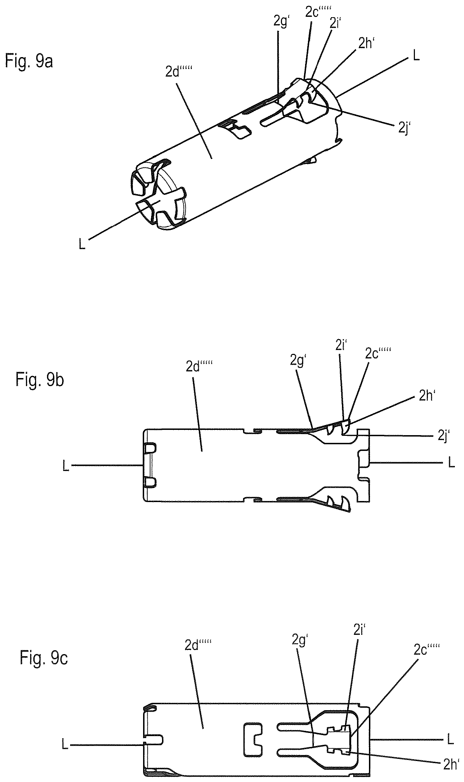

In der Figur 9a ist eine Perspektivenansicht der zweiten Ausführungsform des Eingriffselements (2c''''') mit der Längsachse (L), wobei ein Entfernerelement (2d''''') und das Eingriffselement (2c''''') ersichtlich sind und das Eingriffselement (2c''''') plastisch radial nach aussen verformt ist, dargestellt. Ferner ist in der Figur 9b eine Perspektivenansicht entlang der Längsachse (L) der ersten Ausführungsform des Eingriffselements (2c''''') gemäss Figur 9a ersichtlich. Des Weiteren wird in der Figur 9c eine Perspektivenansicht der ersten Ausführungsform des Eingriffselements (2c''''') gemäss Figur 9b, wobei die Ansicht gegenüber Figur 9b um 90° gedreht ist, gezeigt. Der lange Schenkel (2g') des Eingriffselements (2c''''') ist unter einem Winkel quer zur Längsachse (L) verformbar. In diesem Ausführungsbeispiel ist der lange Schenkel (2g') des Eingriffselements (2c''''') unter einem Winkel von weniger als 90° quer zur Längsachse (L) plastisch radial nach aussen verformt. In einer alternativen Ausführungsform kann der lange Schenkel (2g') des Eingriffselements (2c''''') unter einem Winkel von weniger als 90° quer zur Längsachse (L) plastisch radial nach innen verformt sein. Der kurze Schenkel (2h) ragt radial nach innen.

### Bezugszeichen:

- 1, 1', 1", 1''': Gehäuse
- 1a, 1a', 1a", 1a''': Sperrelement
- 1b, 1b', 1b", 1b''': Gegeneingriffsglied
- 2, 2', 2", 2''': Kappe
- 2a, 2a', 2a", 2a''': Eingriffsglied
- 2b, 2b', 2b", 2b''': Hülse
- 2c, 2c', 2c", 2c''', 2c'''', 2c''''': Eingriffselement
- 2d, 2d', 2d", 2d''', 2d"", 2d''''': Entfernerelement
- 2e, 2e', 2e", 2e''': Vorsprung
- 2f, 2f', 2f", 2f": Ausnehmung
- 2g, 2g': lange Schenkel des Eingriffselements
- 2h, 2h': kurze Schenkel des Eingriffselements
- 2i, 2i': gerade Seite des kurzen Schenkels
- 2j, 2j': Spitze des kurzen Schenkels
- 3, 3', 3", 3''': Produktbehälter
- 3a, 3a', 3a", 3a''': Produktbehälterabschnitt
- 3b: Kolben
- 3c: Flansch
- 3d, 3d', 3d", 3d''': Nadelhalteabschnitt
- 3e, 3e', 3e", 3e''': Injektionsnadel
- 4: Kolbenstange
- 5, 5', 5", 5''': Nadelschutzkappe
- 6, 6', 6", 6''': Produktbehälterhalter
- 7, 7', 7", 7''': Nadelschutzhülse
- 8, 8', 8": Montagewerkzeug
- 8a: Biegeelement
- L: Längsachse

## Patentansprüche

1. Injektionsvorrichtung mit einer Längsachse (L) mit:
- einem Gehäuse (1, 1', 1", 1''') zur Aufnahme eines Produktbehälters (3, 3', 3", 3'''), wobei der Produktbehälter (3, 3', 3", 3''') eine fest verbundene Injektionsnadel (3e, 3e', 3e", 3e''') aufweist, wobei an dem Produktbehälter (3, 3', 3", 3''') eine Nadelschutzkappe (5, 5', 5", 5''') lösbar angeordnet ist, welche die Injektionsnadel (3e, 3e', 3e", 3e''') umschliesst und gegenüber der Umgebung steril abdichtet,
- eine Kappe (2, 2', 2", 2'''), welche lösbar an einem distalen Ende des Gehäuses (1, 1', 1", 1''') vorgesehen ist, wobei die Kappe (2, 2', 2", 2''') ein Eingriffselement (2c, 2c', 2c", 2c''', 2c'''', 2c''''') umfasst, um beim Entfernen der Kappe (2, 2', 2", 2''') von der Injektionsvorrichtung das Entfernen der Nadelschutzkappe (5, 5', 5", 5''') von dem Produktbehälter (3, 3', 3", 3''') zu bewirken,
**dadurch gekennzeichnet, dass** das Eingriffselement (2c, 2c', 2c", 2c''', 2c'''', 2c''''') derart plastisch verformbar ist, dass das Eingriffselement (2c, 2c', 2c", 2c''', 2c'''', 2c''''') von einer beabstandeten Position, in welcher das Eingriffselement (2c, 2c', 2c", 2c''', 2c'''', 2c''''') von der Nadelschutzkappe (5, 5', 5", 5''') radial beabstandet ist, in eine Eingriffsposition, in welcher das Eingriffselement (2c, 2c', 2c", 2c''', 2c'''', 2c''''') in Eingriff mit der Nadelschutzkappe (5, 5', 5", 5''') ist, gelangbar ist, wobei das Eingriffselement (2c, 2c', 2c", 2c''', 2c'''', 2c''''') beim Entfernen der Kappe (2, 2', 2", 2''') plastisch verformt wird.

2. Injektionsvorrichtung nach Anspruch 1,
**dadurch gekennzeichnet, dass** in der beabstandeten Position das Eingriffselement (2c, 2c', 2c", 2c''', 2c'''', 2c''''') unverformt, verformt oder radial nach aussen verformt ist.

3. Injektionsvorrichtung nach Anspruch 1 oder 2,
**dadurch gekennzeichnet, dass** in der Eingriffsposition das Eingriffselement (2c, 2c', 2c", 2c''', 2c'''', 2c''''') unverformt, verformt oder radial nach innen verformt ist.

4. Injektionsvorrichtung nach einem der vorhergehenden Ansprüche,
**dadurch gekennzeichnet, dass** das Eingriffselement (2c, 2c', 2c", 2c''', 2c'''', 2c''''') hakenförmig ausgebildet ist.

5. Injektionsvorrichtung nach Anspruch 4,
**dadurch gekennzeichnet, dass** das hakenförmige Eingriffselement (2c, 2c', 2c", 2c''', 2c'''', 2c''''') einen langen (2g, 2g') und einen kurzen Schenkel (2h, 2h') aufweist, wobei der lange (2g, 2g') und der kurze Schenkel (2h, 2h') miteinander verbunden sind.

6. Injektionsvorrichtung nach Anspruch 5,
**dadurch gekennzeichnet, dass** der lange Schenkel (2g, 2g') des Eingriffselements (2c, 2c', 2c", 2c''', 2c'''', 2c''''') unter einem Winkel, insbesondere unter einem Winkel von weniger als 90° quer zur Längsachse (L) verformbar ist.

7. Injektionsvorrichtung nach Anspruch 5 oder 6,
**dadurch gekennzeichnet, dass** der kurze Schenkel (2h, 2h') des Eingriffselements (2c, 2c', 2c", 2c''', 2c'''', 2c''''') radial nach innen ragt.

8. Injektionsvorrichtung nach einem der Ansprüche 5 bis 7,
**dadurch gekennzeichnet, dass** der kurze Schenkel (2h, 2h') des Eingriffselements (2c, 2c', 2c", 2c''', 2c'''', 2c''''') zahn- oder dreieckförmig oder spitzwinklig ausgebildet ist.

9. Injektionsvorrichtung nach einem der Ansprüche 5 bis 8,
**dadurch gekennzeichnet, dass** sich der lange Schenkel (2g) entlang der Längsachse (L) erstreckt und der lange (2g) und der kurze Schenkel (2h) des Eingriffselements (2c, 2c', 2c", 2c''', 2c'''', 2c''''') derart miteinander verbunden sind, insbesondere plastisch und/oder elastisch verformt verbunden sind, dass sich der kurze Schenkel (2h) von dem langen Schenkel (2g) unter einem Winkel, insbesondere unter einem Winkel von weniger als 90° quer zur Längsachse (L) radial nach innen erstreckt.

10. Injektionsvorrichtung nach einem der Ansprüche 5 bis 8,
**dadurch gekennzeichnet, dass** sich der lange Schenkel (2g') entlang der Längsachse (L) erstreckt und der lange (2g') und der kurze Schenkel (2h') des Eingriffselements (2c, 2c', 2c", 2c''', 2c'''', 2c''''') derart mit einander verbunden sind, insbesondere plastisch und/oder elastisch verformt verbunden sind, dass sich der kurze Schenkel (2h') von dem langen Schenkel (2g') unter einem Winkel, insbesondere unter einem Winkel zur Längsachse (L) entlang der Längsachse (L) radial nach innen erstreckt.

11. Injektionsvorrichtung nach einem der Ansprüche 5 bis 10,
**dadurch gekennzeichnet, dass** an dem langen Schenkel (2g, 2g') mehrere kurze Schenkel (2h, 2h') vorgesehen sind, wobei die mehreren kurzen Schenkel (2h, 2h') um die Längsachse (L) in Umfangsrichtung, quer zur Längsachse (L) und/oder entlang der Längsachse angeordnet sind.

12. Injektionsvorrichtung nach einem der vorhergehenden Ansprüche,
**dadurch gekennzeichnet, dass** das Eingriffselement (2c, 2c', 2c", 2c''', 2c'''', 2c''''') mit der Kappe (2, 2', 2", 2''') axialfest verbunden ist.

13. Injektionsvorrichtung nach einem der vorhergehenden Ansprüche,
**dadurch gekennzeichnet, dass** die Kappe (2, 2', 2", 2''') ferner eine Hülse (2b, 2b', 2b", 2b''') aufweist, wobei die Hülse (2b, 2b', 2b", 2b''') das Eingriffselement (2c, 2c', 2c", 2c''', 2c'''', 2c''''') zumindest teilweise umfasst und dass, das Eingriffselement (2c, 2c', 2c", 2c''', 2c'''', 2c''''') mit der Hülse (2b, 2b', 2b", 2b''') axialfest verbunden ist.

14. Injektionsvorrichtung nach einem der vorhergehenden Ansprüche,
**dadurch gekennzeichnet, dass** an dem Gehäuse (1, 1', 1", 1''') oder an einem Gehäuse (1, 1', 1", 1''') fest verbundenen Teil ein Sperrelement (1a, 1a', 1a", 1a''') vorgesehen ist, wobei in der Eingriffsposition des Eingriffselements (2c, 2c', 2c", 2c''', 2c'''', 2c''''') das Sperrelement (1a, 1a' 1a", 1a''') das Eingriffselement (2c, 2c', 2c", 2c''', 2c'''', 2c''''') in Eingriff mit der Nadelschutzkappe (5, 5', 5", 5''') hält oder bringt.

15. Verfahren zum Montieren einer Injektionsvorrichtung und/oder Vorbereiten einer Injektionsvorrichtung für die Verabreichung eines Produkts nach einem der vorhergehenden Ansprüche mit folgenden Schritten:
- Bereitstellen eines Gehäuses (1, 1', 1", 1''') zur Aufnahme eines Produktbehälters (3, 3', 3", 3'''),
- Bereitstellen einer Kappe (2, 2', 2", 2'''), welche an einem distalen Ende des Gehäuses (1, 1', 1", 1''') lösbar angebracht wird, wobei die Kappe (2, 2', 2", 2''') ein Eingriffselement (2c, 2c', 2c", 2c''', 2c'''', 2c''''') umfasst, um beim Entfernen der Kappe (2, 2', 2", 2''') von der Injektionsvorrichtung das Entfernen der Nadelschutzkappe (5, 5', 5", 5''') von dem Produktbehälter (3, 3', 3", 3''') zu bewirken,
- Anbringen der Kappe (2, 2', 2", 2''') an dem distalen Ende des Gehäuses (1, 1', 1", 1'''),
- Bereitstellen eines Produktbehälters (3, 3', 3", 3'''), der eine fest verbundene Injektionsnadel (3e, 3e', 3e", 3e''') aufweist, wobei an dem Produktbehälter (3, 3', 3", 3''') eine Nadelschutzkappe (5, 5', 5", 5''') lösbar angeordnet ist, welche die Injektionsadel (3e, 3e', 3e", 3e''') umschliesst und gegenüber der Umgebung steril abdichtet, **gekennzeichnet durch**
- Verschieben oder Einsetzen des Produktbehälters (3, 3', 3", 3''') mit der lösbar verbundenen Nadelschutzkappe (5, 5', 5", 5''') in das Gehäuse (1, 1', 1", 1''') entlang einer Längsachse (L) in eine distale Richtung, wobei das Eingriffselement (2c, 2c', 2c", 2c''', 2c'''', 2c''''') derart plastisch verformbar, verformt oder vorverformt ist, dass beim Verschieben des Produktbehälters (3, 3', 3", 3''') relativ zu dem Gehäuse (1, 1', 1", 1''') in die distale Richtung die Nadelschutzkappe (5, 5', 5", 5''') von dem Eingriffselement (2c, 2c', 2c", 2c''', 2c'''', 2c''''') radial beabstandet ist.

16. Verfahren nach Anspruch 15,
**dadurch gekennzeichnet, dass** das Eingriffselement (2c, 2c', 2c", 2c''', 2c'''', 2c''''') derart plastisch verformbar ist, dass während dem Entfernen der Kappe (2, 2', 2", 2''') von der Injektionsvorrichtung das Eingriffselement (2c, 2c', 2c", 2c''', 2c'''', 2c''''') in Eingriff mit der Nadelschutzkappe (5, 5', 5", 5''') gebracht wird.

17. Verfahren nach Anspruch 16,
**dadurch gekennzeichnet, dass** an dem Gehäuse (1, 1', 1", 1''') oder an einem Gehäuse (1, 1', 1", 1''') fest verbundenen Teil ein Sperrelement (1a, 1a', 1a", 1a''') vorgesehen ist, welches das Eingriffselement (2c, 2c', 2c", 2c''', 2c'''', 2c''''') in Eingriff mit der Nadelschutzkappe (5, 5', 5", 5''') bringt.

18. Verfahren nach einem der Ansprüche 15 bis 17,
**dadurch gekennzeichnet, dass** vor dem Verschieben des Produktbehälters (3, 3', 3", 3''') relativ zu dem Gehäuse (1, 1', 1", 1''') das Eingriffselement (2c, 2c', 2c", 2c''', 2c'''', 2c''''') unverformt, verformt oder radial nach aussen verformt wird.

19. Verfahren nach Anspruch 18,
**dadurch gekennzeichnet, dass** ein Montagewerkzeug (8, 8', 8", 8''') verwendet wird, um das Eingriffselement (2c, 2c', 2c", 2c''', 2c'''', 2c''''') in eine beabstandete Position, in welcher das Eingriffselement (2c, 2c', 2c", 2c''') von der Nadelschutzkappe (5, 5', 5", 5''') radial beabstandet ist, zu bringen.

20. Verfahren nach einem der Ansprüche 15 bis 19,
**dadurch gekennzeichnet, dass** eine Nadelschutzhülse (7, 7', 7", 7''') zumindest teilweise in das Gehäuse (1, 1', 1", 1''') gesetzt wird, wobei an einem distalen Ende der Nadelschutzhülse (7, 7', 7", 7''') die Kappe (2, 2', 2", 2''') aufgesetzt wird.

## Claims

1. Injection device having a longitudinal axis (L), comprising:
- a housing (1, 1', 1", 1''') for receiving a product container (3, 3', 3", 3'''), wherein the product container (3, 3', 3", 3''') has a fixedly connected injection needle (3e, 3e', 3e", 3e'''), wherein a needle protection cap (5, 5', 5", 5''') that encloses the injection needle (3e, 3e', 3e", 3e''') and seals it sterilely against the surroundings is releasably arranged on the product container (3, 3', 3", 3'''),
- a device cap (2, 2', 2", 2''') that is releasably provided at the distal end of the housing (1, 1', 1", 1'''), wherein the device cap (2, 2', 2", 2''') comprises an engagement element (2c, 2c', 2c", 2c''', 2c'''', 2c''''') in order to effect the removal of the needle protection cap (5, 5', 5", 5''') from the product container (3, 3', 3", 3''') when the device cap (2, 2', 2", 2''') is removed from the injection device,
**characterized in that** the engagement element (2c, 2c', 2c", 2c''', 2c'''', 2c''''') is deformable plastically in such a manner that the engagement element (2c, 2c', 2c", 2c''', 2c'''', 2c''''') can move from a spaced-apart position, in which the engagement element (2c, 2c', 2c", 2c''', 2c'''', 2c''''') is at a radial distance from the needle protection cap (5, 5', 5", 5'''), into an engagement position, in which the engagement element (2c, 2c', 2c", 2c''', 2c'''', 2c''''') is engaged with the needle protection cap (5, 5', 5", 5'''), wherein the engagement element (2c, 2c', 2c", 2c''', 2c'''', 2c''''') is deformed plastically during removal of the device cap (2, 2', 2", 2''').

2. Injection device according to Claim 1,
**characterized in that** the engagement element (2c, 2c', 2c", 2c''', 2c'''', 2c''''') is undeformed, deformed or deformed radially outwardly in the spaced-apart position.

3. Injection device according to Claim 1 or 2,
**characterized in that** the engagement element (2c, 2c', 2c", 2c''', 2c'''', 2c''''') is undeformed, deformed or deformed radially inwardly in the engagement position.

4. Injection device according to one of the preceding claims,
**characterized in that** the engagement element (2c, 2c', 2c", 2c''', 2c'''', 2c''''') is hook-shaped.

5. Injection device according to Claim 4,
**characterized in that** the hook-shaped engagement element (2c, 2c', 2c", 2c''', 2c'''', 2c''''') has a long limb (2g, 2g') and a short limb (2h, 2h'), the long limb (2g, 2g') and the short limb (2h, 2h') being connected to one another.

6. Injection device according to Claim 5,
**characterized in that** the long limb (2g, 2g') of the engagement element (2c, 2c', 2c", 2c''', 2c'''', 2c''''') can be deformed at an angle, more particularly an angle of less than 90°, transverse to the longitudinal axis (L).

7. Injection device according to Claim 5 or 6,
**characterized in that** the short limb (2h, 2h') of the engagement element (2c, 2c', 2c", 2c''', 2c'''', 2c''''') protrudes radially inwardly.

8. Injection device according to one of Claims 5-7,
**characterized in that** the short limb (2h, 2h') of the engagement element (2c, 2c', 2c", 2c''', 2c'''', 2c''''') is tooth-shaped, triangular or acute-angled.

9. Injection device according to one of Claims 5-8,
**characterized in that** the long limb (2g) extends along the longitudinal axis (L), and the long limb (2g) and the short limb (2h) of the engagement element (2c, 2c', 2c", 2c''', 2c'''', 2c''''') are connected to one another, in particular connected with a plastic or elastic deformation, such that the short limb (2h) extends from the long limb (2g) radially inwardly at an angle, more particularly at an angle of less than 90°, transverse to the longitudinal axis (L).

10. Injection device according to one of Claims 5-8,
**characterized in that** the long limb (2g') extends along the longitudinal axis (L), and the long limb (2g') and the short limb (2h') of the engagement element (2c, 2c', 2c", 2c''', 2c'''', 2c''''') are connected to one another, more particularly connected with a plastic or elastic deformation, such that the short limb (2h') extends radially inwardly from the long limb (2g') at an angle, more particularly an angle relative to the longitudinal axis (L), along the longitudinal axis (L).

11. Injection device according to one of Claims 5-10,
**characterized in that** multiple short limbs (2h, 2h') are provided on the long limb (2g, 2g'), wherein the multiple short limbs (2h, 2h') are arranged in the circumferential direction about the longitudinal axis (L), transverse to the longitudinal axis (L) and/or along the longitudinal axis.

12. Injection device according to one of the preceding claims,
**characterized in that** the engagement element (2c, 2c', 2c", 2c''', 2c'''', 2c''''') is axially fixedly connected to the device cap (2, 2', 2", 2''').

13. Injection device according to one of the preceding claims,
**characterized in that** the device cap (2, 2', 2", 2''') additionally has a sleeve (2b, 2b', 2b", 2b'''), wherein the sleeve (2b, 2b', 2b", 2b''') at least partially surrounds the engagement element (2c, 2c', 2c", 2c''', 2c'''', 2c''''') and **in that** the engagement element (2c, 2c', 2c", 2c''', 2c'''', 2c''''') is axially fixedly connected to the sleeve (2b, 2b', 2b", 2b''').

14. Injection device according to one of the preceding claims,
**characterized in that** a blocking element (1a, 1a', 1a", 1a''') is provided on the housing (1, 1', 1", 1''') or on a part fixedly connected to the housing (1, 1', 1", 1'''), the blocking element (1a, 1a' 1a", 1a''') bringing the engagement element (2c, 2c', 2c", 2c''', 2c'''', 2c''''') into engagement with the needle protection cap (5, 5', 5", 5''') in the engagement position of the engagement element (2c, 2c', 2c", 2c''', 2c'''', 2c) or keeping it engaged.

15. Method for assembling an injection device and/or preparing an injection device for the administration of a product, comprising the following steps:
- providing a housing (1, 1', 1", 1''') for receiving a product container (3, 3', 3", 3'''),
- providing a device cap (2, 2', 2", 2''') that is releasably attached at the distal end of the housing (1.1, 1', 1", 1'''), wherein the device cap (2, 2', 2", 2''') comprises an engagement element (2c, 2c', 2c", 2c''', 2c'''', 2c'''''') in order to effect the removal of the needle protection cap (5, 5', 5", 5''') from the product container (3, 3', 3", 3''') when the device cap (2, 2', 2", 2''') is being removed from the injection device,
- attaching the device cap (2, 2', 2", 2''') at the distal end of the housing (1, 1', 1", 1'''),
- providing a product container (3, 3', 3", 3'''), having a fixedly connected injection needle (3e, 3e', 3e", 3e'''), wherein a needle protection cap (5, 5', 5", 5''') that encloses the injection needle (3e, 3e', 3e", 3e''') and seals it sterilely against the surroundings is releasably arranged on the product container (3, 3', 3", 3'''),
**characterized by**
- displacing or inserting the product container (3, 3', 3", 3''') having the releasably connected needle protection cap (5, 5', 5", 5''') into the housing (1, 1', 1", 1''') in a distal direction along a longitudinal axis (L), wherein the engagement element (2c, 2c', 2c", 2c''', 2c'''', 2c''''') is plastically deformable, deformed or pre-deformed such that, during displacement of the product container (3, 3', 3", 3''') in the distal direction relative to the housing (1, 1', 1", 1'''), the needle protection cap (5, 5', 5", 5''') is at a radial distance from the engagement element (2c, 2c', 2c", 2c''', 2c'''', 2c''''').

16. Method according to Claim 15,
**characterized in that** the engagement element (2c, 2c', 2c", 2c''', 2c'''', 2c) can be deformed plastically such that the engagement element (2c, 2c', 2c", 2c''', 2c'''', 2c''''') is brought into engagement with the needle protection cap (5, 5', 5", 5''') during the removal of the device cap (2, 2', 2", 2''') from the injection device.

17. Method according to Claim 16,
**characterized in that** a blocking element (1a, 1a', 1a", 1a''') is provided on a housing (1, 1', 1", 1''') or a part fixedly connected to the housing (1, 1', 1", 1''') and brings the engagement element (2c, 2c', 2c", 2c''', 2c'''', 2c''''') into engagement with the needle protection cap (5, 5', 5", 5''').

18. Method according to one of Claims 15-17,
**characterized in that** the engagement element (2c, 2c', 2c", 2c''', 2c'''', 2c''''') is undeformed, deformed or deformed radially outwardly before displacement of the product containers (3, 3', 3", 3''') relative to the housings (1, 1', 1", 1''').

19. Method according to Claim 18,
**characterized in that** an assembly tool (8, 8', 8", 8''') is used to bring the engagement element (2c, 2c', 2c", 2c''', 2c'''', 2c) into a spaced-apart position, in which the engagement element (2c, 2c', 2c", 2c''') is at a radial distance from the needle protection cap (5, 5', 5", 5''').

20. Method according to one of Claims 15-19,
**characterized in that** a needle protection sleeve (7, 7', 7", 7''') is inserted at least in part into the housing (1, 1', 1", 1'''), wherein the device cap (2, 2, 2", 2''') is placed at a distal end of the needle protection sleeve (7, 7', 7", 7''').

## Revendications

1. Dispositif d'injection avec un axe longitudinal (L) avec :
- un boîtier (1, 1', 1", 1''') pour loger un contenant de produit (3, 3', 3", 3'''), dans lequel le contenant de produit (3, 3', 3", 3''') présente une aiguille d'injection (3e, 3e', 3e", 3e''') reliée de manière fixe, dans lequel un capuchon de protection d'aiguille (5, 5', 5", 5'''), lequel entoure l'aiguille d'injection (3e, 3e', 3e", 3e"') et l'étanchéifie de manière stérile par rapport à l'environnement, est disposé de manière détachable sur le contenant de produit (3, 3', 3", 3'''),
- un capuchon (2, 2', 2", 2'''), lequel est prévu de manière détachable à une extrémité distale du boîtier (1, 1', 1", 1'''), dans lequel le capuchon (2, 2', 2", 2''') comprend un élément d'engagement (2c, 2c', 2c", 2c''', 2c'''', 2c'''''), afin, lors du retrait du capuchon (2, 2', 2", 2''') du dispositif d'injection, de provoquer le retrait du capuchon de protection d'aiguille (5, 5', 5", 5''') du contenant de produit (3, 3', 3", 3'''),
**caractérisé en ce que** l'élément d'engagement (2c, 2c', 2c", 2c''', 2c'''', 2c''''') peut être déformé plastiquement de telle sorte que l'élément d'engagement (2c, 2c', 2c", 2c''', 2c'''', 2c''''') peut parvenir à partir d'une position éloignée, dans laquelle l'élément d'engagement (2c, 2c', 2c", 2c''', 2c'''', 2c''''') est éloigné radialement du capuchon de protection d'aiguille (5, 5', 5", 5'''), dans une position d'engagement, dans laquelle l'élément d'engagement (2c, 2c', 2c", 2c''', 2c'''', 2c''''') est en engagement avec le capuchon de protection d'aiguille (5, 5', 5", 5'''), dans lequel l'élément d'engagement (2c, 2c', 2c", 2c''', 2c'''', 2c''''') est déformé plastiquement lors du retrait du capuchon (2, 2', 2", 2''').

2. Dispositif d'injection selon la revendication 1,
**caractérisé en ce que**, dans la position éloignée, l'élément d'engagement (2c, 2c', 2c", 2c'", 2c'''', 2c''''') est non déformé, déformé ou déformé radialement vers l'extérieur.

3. Dispositif d'injection selon la revendication 1 ou 2,
**caractérisé en ce que**, dans la position d'engagement, l'élément d'engagement (2c, 2c', 2c", 2c''', 2c'''', 2c""') est non déformé, déformé ou déformé radialement vers l'intérieur.

4. Dispositif d'injection selon l'une quelconque des revendications précédentes, **caractérisé en ce que** l'élément d'engagement (2c, 2c', 2c", 2c'", 2c'''', 2c''''') est réalisé en forme de crochet.

5. Dispositif d'injection selon la revendication 4,
**caractérisé en ce que** l'élément d'engagement (2c, 2c', 2c", 2c''', 2c'''', 2c''''') en forme de crochet présente une branche longue (2g, 2g') et une branche courte (2h, 2h'), dans lequel la branche longue (2g, 2g') et la branche courte (2h, 2h') sont reliées l'une à l'autre.

6. Dispositif d'injection selon la revendication 5,
**caractérisé en ce que** la branche longue (2g, 2g') de l'élément d'engagement (2c, 2c', 2c", 2c''', 2c'''', 2c''''') peut être déformée selon un angle, en particulier selon un angle de moins de 90° transversalement par rapport à l'axe longitudinal (L).

7. Dispositif d'injection selon la revendication 5 ou 6,
**caractérisé en ce que** la branche courte (2h, 2h') de l'élément d'engagement (2c, 2c', 2c", 2c''', 2c'''', 2c''''') fait saillie radialement vers l'intérieur.

8. Dispositif d'injection selon l'une quelconque des revendications 5 à 7,
**caractérisé en ce que** la branche courte (2h, 2h') de l'élément d'engagement (2c, 2c', 2c", 2c''', 2c'''', 2c''''') est réalisée en forme de dent ou de triangle ou à angle aigu.

9. Dispositif d'injection selon l'une quelconque des revendications 5 à 8,
**caractérisé en ce que** la branche longue (2g) s'étend le long de l'axe longitudinal (L) et la branche longue (2g) et la branche courte (2h) de l'élément d'engagement (2c, 2c', 2c", 2c''', 2c'''', 2c''''') sont reliées l'une à l'autre, en particulier reliées de manière déformée plastiquement et/ou élastiquement, de telle sorte que la branche courte (2h) s'étend à partir de la branche longue (2g) selon un angle, en particulier selon un angle de moins de 90° transversalement par rapport à l'axe longitudinal (L) radialement vers l'intérieur.

10. Dispositif d'injection selon l'une quelconque des revendications 5 à 8,
**caractérisé en ce que** la branche longue (2g') s'étend le long de l'axe longitudinal (L) et la branche longue (2g') et la branche courte (2h') de l'élément d'engagement (2c, 2c', 2c", 2c''', 2c"", 2c''''') sont reliées l'une à l'autre, en particulier reliées de manière déformée plastiquement et/ou élastiquement, de telle sorte que la branche courte (2h') s'étend à partir de la branche longue (2g') selon un angle, en particulier selon un angle par rapport à l'axe longitudinal (L) le long de l'axe longitudinal (L) radialement vers l'intérieur.

11. Dispositif d'injection selon l'une quelconque des revendications 5 à 10,
**caractérisé en ce que** plusieurs branches courtes (2h, 2h') sont prévues sur la branche longue (2g, 2g'), dans lequel les plusieurs branches courtes (2h, 2h') sont disposées autour de l'axe longitudinal (L) dans la direction périphérique, transversalement par rapport à l'axe longitudinal (L) et/ou le long de l'axe longitudinal.

12. Dispositif d'injection selon l'une quelconque des revendications précédentes,
**caractérisé en ce que** l'élément d'engagement (2c, 2c', 2c", 2c'", 2c'''', 2c''''') est relié de manière axialement fixe au capuchon (2, 2', 2", 2''').

13. Dispositif d'injection selon l'une quelconque des revendications précédentes,
**caractérisé en ce que** le capuchon (2, 2', 2", 2"') présente en outre un manchon (2b, 2b', 2b", 2b'''), dans lequel le manchon (2b, 2b', 2b", 2b''') entoure au moins en partie l'élément d'engagement (2c, 2c', 2c", 2c''', 2c"", 2c''''') et que l'élément d'engagement (2c, 2c', 2c", 2c''', 2c'''', 2c''''') est relié au manchon (2b, 2b', 2b", 2b''') de manière axialement fixe.

14. Dispositif d'injection selon l'une quelconque des revendications précédentes,
**caractérisé en ce qu'**un élément de verrouillage (1a, la', la", 1a''') est prévu sur le boîtier (1, 1', 1", 1''') ou sur une partie reliée fixement à un boîtier (1, 1', 1", 1'''), dans lequel, dans la position d'engagement de l'élément d'engagement (2c, 2c', 2c", 2c''', 2c'''', 2c'''''), l'élément de verrouillage (1a, 1a', 1a", 1a''') retient ou amène l'élément d'engagement (2c, 2c', 2c", 2c''', 2c'''', 2c''''') en engagement avec le capuchon de protection d'aiguille (5, 5', 5", 5"').

15. Procédé pour monter un dispositif d'injection et/ou préparer un dispositif d'injection pour l'administration d'un produit selon l'une quelconque des revendications précédentes avec les étapes suivantes :
- la fourniture d'un boîtier (1, 1', 1", 1''') pour loger un contenant de produit (3, 3', 3", 3'''),
- la fourniture d'un capuchon (2, 2', 2", 2'''), lequel est fixé de manière détachable à une extrémité distale du boîtier (1, 1', 1", 1'''), dans lequel le capuchon (2, 2', 2", 2''') comprend un élément d'engagement (2c, 2c', 2c", 2c''', 2c'''', 2c'''''), afin, lors du retrait du capuchon (2, 2', 2", 2''') du dispositif d'injection, de provoquer le retrait du capuchon de protection d'aiguille (5, 5', 5", 5''') du contenant de produit (3, 3', 3", 3'''),
- la fixation du capuchon (2, 2', 2", 2''') à l'extrémité distale du boîtier (1, 1', 1", 1'''),
- la fourniture d'un contenant de produit (3, 3', 3", 3'''), qui présente une aiguille d'injection (3e, 3e', 3e", 3e"') reliée de manière fixe, dans lequel un capuchon de protection d'aiguille (5, 5', 5", 5'''), lequel entoure l'aiguille d'injection (3e, 3e', 3e", 3e''') et l'étanchéifie de manière stérile par rapport à l'environnement, est disposé de manière détachable sur le contenant de produit (3, 3', 3", 3'''),
**caractérisé par**
- le déplacement ou l'insertion du contenant de produit (3, 3', 3", 3''') avec le capuchon de protection d'aiguille (5, 5', 5", 5''') relié de manière détachable dans le boîtier (1, 1', 1", 1''') le long d'un axe longitudinal (L) dans une direction distale, dans lequel l'élément d'engagement (2c, 2c', 2c", 2c''', 2c'''', 2c''''') est déformable, déformé ou pré-déformé plastiquement, de telle sorte que, lors du déplacement du contenant de produit (3, 3', 3", 3"') par rapport au boîtier (1, 1', 1", 1''') dans la direction distale, le capuchon de protection d'aiguille (5, 5', 5", 5''') est éloigné radialement de l'élément d'engagement (2c, 2c', 2c", 2c''', 2c'''', 2c''''').

16. Procédé selon la revendication 15,
**caractérisé en ce que** l'élément d'engagement (2c, 2c', 2c", 2c'", 2c'''', 2c''''') est déformable plastiquement de telle sorte que, pendant le retrait du capuchon (2, 2', 2", 2''') du dispositif d'injection, l'élément d'engagement (2c, 2c', 2c", 2c'", 2c'''', 2c''''') est amené en engagement avec le capuchon de protection d'aiguille (5, 5', 5", 5''').

17. Procédé selon la revendication 16,
**caractérisé en ce qu'**un élément de verrouillage (1a, 1a', 1a", 1a'''), lequel amène l'élément d'engagement (2c, 2c', 2c", 2c''', 2c'''', 2c''''') en engagement avec le capuchon de protection d'aiguille (5, 5', 5", 5"'), est prévu sur le boîtier (1, 1', 1", 1''') ou sur une partie reliée fixement à un boîtier (1, 1', 1", 1''').

18. Procédé selon l'une quelconque des revendications 15 à 17,
**caractérisé en ce que**, avant le déplacement du contenant de produit (3, 3', 3", 3''') par rapport au boîtier (1, 1', 1", 1'''), l'élément d'engagement (2c, 2c', 2c", 2c''', 2c'''', 2c''''') est non déformé, déformé ou déformé radialement vers l'extérieur.

19. Procédé selon la revendication 18,
**caractérisé en ce qu'**un outil de montage (8, 8', 8", 8''') est utilisé, afin d'amener l'élément d'engagement (2c, 2c', 2c", 2c''', 2c'''', 2c''''') dans une position éloignée, dans laquelle l'élément d'engagement (2c, 2c', 2c", 2c''') est radialement éloigné du capuchon de protection d'aiguille (5, 5', 5", 5"').

20. Procédé selon l'une quelconque des revendications 15 à 19,
**caractérisé en ce qu'**un manchon de protection d'aiguille (7, 7', 7", 7''') est posé au moins en partie dans le boîtier (1, 1', 1", 1'''), dans lequel le capuchon (2, 2', 2", 2"') est mis en place à une extrémité distale du manchon de protection d'aiguille (7, 7', 7", 7''').
